# EUROPEAN PATENT APPLICATION

(11) **EP 4 299 756 A1**
(43) Date of publication of application: **03.01.2024**
(21) Application number: 22767112.0
(22) Date of filing: 08.03.2022
(51) Int. Cl.: C12P 21/02, C07K 1/16, C07K 16/28, C07K 19/00, C12N 9/24, C12N 15/13, C12N 15/56, C12N 15/62

(54) **METHOD FOR PRODUCING ANTIBODY-LYSOSOMAL ENZYME FUSION PROTEIN**

(30) Priority: 09.03.2021 JP 2021037528
(71) Applicant: JCR Pharmaceuticals Co., Ltd., Ashiya-shi, Hyogo 659-0021 (JP)
(72) Inventor: KAKIMOTO, Shinji, Kobe-shi, Hyogo 651-2241 (JP); FUKUI, Tsuyoshi, Kobe-shi, Hyogo 651-2241 (JP); HATANO, Yukichi, Kobe-shi, Hyogo 651-2241 (JP); KOTANI, Ayaka, Kobe-shi, Hyogo 651-2241 (JP); MIURA, Takuya, Kobe-shi, Hyogo 651-2241 (JP); ISHIGURO, Toshifumi, Kobe-shi, Hyogo 651-2241 (JP)
(74) Representative: Boult Wade Tennant LLP
(86) International application number: PCT/JP2022/009851
(87) International publication number: WO 2022/191158

(57) **Abstract**

Disclosed is a method for producing a fusion protein in which an antibody and a lysosomal enzyme are fused. The method is a method for producing a fusion protein in which an antibody and a human lysosomal enzyme are fused, the method including: (a) culturing mammalian cells producing the fusion protein in a serum-free medium to secrete the fusion protein in a culture medium; (b) collecting a culture supernatant by removing the mammalian cells from the culture medium; and (c) purifying the fusion protein from the culture supernatant by using a column chromatography using a material to which a substance having affinity for the antibody is bound as a solid phase, an anion exchange column chromatography, a cation exchange column chromatography, and a size exclusion column chromatography.

## Description

### Technical Field

The present invention relates to a method for producing a fusion protein in which an antibody and a lysosomal enzyme are fused, for example, a method for purifying a recombinant fusion protein obtained by culturing a host cell into which an expression vector incorporating a gene encoding the fusion protein is introduced to a purity at which the recombinant fusion protein can be used as medical drugs.

### Background Art

Currently, many medical drugs containing a recombinant protein as an active ingredient have been commercially available. Such a recombinant protein is obtained in a culture supernatant by culturing a host cell to which an expression vector incorporating a gene encoding the protein of interest is introduced. The recombinant protein obtained in the culture supernatant is not capable of being used as medical drugs as it is, because the recombinant protein contains contaminants. In order to be used as medical drugs, it is necessary to purify the recombinant protein in the culture supernatant.

A method for purifying a recombinant protein obtained in a culture supernatant by using mammalian cells as a host cell and culturing the host cell to a level at which the recombinant protein can be used as medical drugs has been reported. For example, a method has been reported in which human erythropoietin (hEPO), which is glycoprotein acting on erythroblast progenitor cells to differentiate the erythroblast progenitor cells to erythrocytes and promote the production of the erythrocytes, is expressed as a recombinant protein by using CHO cells as a host cell, and is purified from a culture supernatant by using various chromatographies including a dye affinity column chromatography until the human erythropoietin can be used as medical drugs (Patent Literature 1). In addition, for example, a method has been reported in which human follicle stimulating hormone (hFSH), which is one type of gonadotropic hormones having activity to promote the production and the secretion of estrogen in the ovary, is expressed as a recombinant protein by using CHO cells as a host cell, and is purified from a culture supernatant by using various chromatographies including a cation exchange column chromatography until the human follicle stimulating hormone can be used as medical drugs (Patent Literature 2). In addition, for example, a method has been reported in which human iduronate-2-sulfatase (hI2S), which is one type of lysosome enzyme having activity of hydrolyzing a sulfate bond in glycosaminoglycan (GAG) molecules such as heparan sulfate or dermatan sulfate, is expressed as a recombinant protein by using CHO cells as a host cell, and is purified from a culture supernatant by using various chromatographies including a cation exchange column chromatography until the human iduronate-2-sulfatase can be used as medical drugs (Patent Literature 3). In addition, for example, a method has been reported in which human α-galactosidase A (ha-Gal A), which is one type of lysosome enzyme having activity of hydrolyzing a terminal α-galactosyl bond of glycolipid and glycoprotein, is expressed as a recombinant protein by using CHO cells as a host cell, and is purified from a culture supernatant by using various chromatographies including an anion exchange column chromatography until the human α-galactosidase A can be used as medical drugs (Patent Literature 4 and Patent Literature 5). Further, for example, a method has been reported in which human DNaseI having activity of nonspecifically degrading DNA in a base sequence is expressed as a recombinant protein by using CHO cells as a host cell, and is purified from a culture supernatant by using various chromatographies including an anion exchange column chromatography and a dye ligand affinity column chromatography until the human DNaseI can be used as medical drugs (Patent Literature 6). Further, for example, a method has been reported in which an anti-hTfR antibody bound to human iduronate-2-sulfatase is expressed as a recombinant protein by using CHO cells as a host cell, and is purified from a culture supernatant by using a protein A affinity column chromatography, a hydroxyapatite column chromatography, and a size exclusion column chromatography until the anti-hTfR antibody can be used as medical drugs (Patent Literature 7).

As described above, in order to acquire a recombinant protein that can be used as medical drugs, a unique purification method has been developed for each of the recombinant proteins.

### Citation List

### Patent Literature

Patent Literature 1: Japanese Unexamined Patent Publication No. 2010-511378
Patent Literature 2: Japanese Unexamined Patent Publication No. 2009-273427
Patent Literature 3: Japanese Unexamined Patent Publication No. 2014-508506
Patent Literature 4: International Publication WO 2014/017088
Patent Literature 5: International Publication WO 2016/117341
Patent Literature 6: International Publication WO 2016/067944
Patent Literature 7: International Publication WO 2018/038243

### Summary of Invention

### Technical Problem

An object of the present invention is to provide a method for expressing a fusion protein in which an antibody and a lysosomal enzyme are fused as a recombinant protein and purifying the fusion protein to a purity at which the fusion protein can be distributed to the market as medical drugs.

### Solution to Problem

In studies for the object described above, as a result of intensive studies of the present inventors, it has been found that a fusion protein in which an anti-transferrin receptor antibody and human α-L-iduronidase (hIDUA) are fused can be efficiently purified at a high purity by culturing mammalian cells to which an expression vector incorporating a gene encoding the fusion protein is introduced in a serum-free medium, and purifying the fusion protein obtained in a culture medium in accordance with a combination of a column chromatography using a material to which a substance having affinity for the antibody is bound as a solid phase, an anion exchange column chromatography, a cation exchange column chromatography, and a size exclusion column chromatography. The present invention has been completed on the basis of such findings and with further studies. That is, the present invention provides the followings.
1. A method for producing a fusion protein in which an antibody and a human lysosomal enzyme are fused, the method including:
   (a) a step of culturing mammalian cells producing the fusion protein in a serum-free medium to secrete the fusion protein in a culture medium;
   (b) a step of collecting a culture supernatant by removing the mammalian cells from the culture medium obtained in the step (a); and
   (c) a step of purifying the fusion protein from the culture supernatant obtained in the step (b) by using a column chromatography using a material to which a substance having affinity for the antibody is bound as a solid phase, an anion exchange column chromatography, a cation exchange column chromatography, and a size exclusion column chromatography.
2. The method for producing a fusion protein according to 1 described above, in which in the step (c), the column chromatography using the material to which the substance having affinity for the antibody is bound as the solid phase, the anion exchange column chromatography, the cation exchange column chromatography, and the size exclusion column chromatography are used in this order.
3. The method for producing a fusion protein according to 1 or 2 described above, in which the substance having affinity for the antibody has affinity for a CH₁ region of a heavy chain of the antibody.
4. The method for producing a fusion protein according to any one of 1 to 3 described above, in which an anion exchanger used in the anion exchange column chromatography is a strong anion exchanger.
5. The method for producing a fusion protein according to any one of 1 to 4 described above, in which a cation exchanger used in the cation exchange column chromatography is a weak cation exchanger.
6. The method for producing a fusion protein according to any one of 1 to 5 described above, in which the antibody fused with the human lysosomal enzyme is a humanized antibody or a human antibody.
7. The method for producing a fusion protein according to any one of 1 to 5 described above, in which the antibody fused with the human lysosomal enzyme is a humanized antibody.
8. The method for producing a fusion protein according to any one of 1 to 7 described above, in which the antibody fused with the human lysosomal enzyme recognizes a molecule present on a surface of vascular endothelial cells as an antigen.
9. The method for producing a fusion protein according to 8 described above, in which the molecule present on the surface of the vascular endothelial cells is selected from the group consisting of a transferrin receptor (TfR), an insulin receptor, a leptin receptor, a lipoprotein receptor, an IGF receptor, OATP-F, an organic anion transporter, MCT-8, a monocarboxylic acid transporter, and a Fc receptor.
10. The method for producing a fusion protein according to 8 described above, in which the vascular endothelial cells are cerebral vascular endothelial cells.
11. The method for producing a fusion protein according to 10 described above, in which a molecule present on a surface of the cerebral vascular endothelial cells is selected from the group consisting of a transferrin receptor (TfR), an insulin receptor, a leptin receptor, a lipoprotein receptor, an IGF receptor, OATP-F, an organic anion transporter, MCT-8, and a monocarboxylic acid transporter.
12. The method for producing a fusion protein according to any one of 8 to 11 described above, in which the vascular endothelial cells are human vascular endothelial cells.
13. The method for producing a fusion protein according to any one of 1 to 12 described above, in which the antibody is an anti-human transferrin receptor antibody,
   in a variable region of a heavy chain of the antibody, CDR1 includes an amino acid sequence of SEQ ID NO: 13 or SEQ ID NO: 14, CDR2 includes an amino acid sequence of SEQ ID NO: 15 or SEQ ID NO: 16, and CDR3 includes an amino acid sequence of SEQ ID NO: 17 or SEQ ID NO: 18, and
   in a variable region of a light chain of the antibody, CDR1 includes an amino acid sequence of SEQ ID NO:8 or SEQ ID NO:9, CDR2 includes an amino acid sequence of SEQ ID NO: 10 or SEQ ID NO: 11, or an amino acid sequence Lys-Val-Ser, and CDR3 includes an amino acid sequence of SEQ ID NO: 12.
14. The method for producing a fusion protein according to 13 described above, in which a framework region 3 of the heavy chain of the antibody includes an amino acid sequence of SEQ ID NO: 19.
15. The method for producing a fusion protein according to 14 described above, in which the variable region of the heavy chain in the antibody includes an amino acid sequence of SEQ ID NO:21.
16. The method for producing a fusion protein according to 13 or 14 described above, in which in the variable region of the heavy chain, the antibody includes an amino acid sequence having 80% or more of identity with an amino acid sequence of SEQ ID NO:21,
   CDR1 includes an amino acid sequence of SEQ ID NO: 13 or SEQ ID NO: 14,
   CDR2 includes an amino acid sequence of SEQ ID NO: 15 or SEQ ID NO: 16,
   CDR3 includes an amino acid sequence of SEQ ID NO: 17 or SEQ ID NO: 18, and
   the framework region 3 includes an amino acid sequence of SEQ ID NO: 19.
17. The method for producing a fusion protein according to 13 or 14 described above, in which in the variable region of the heavy chain, the antibody includes an amino acid sequence having 90% or more of identity with an amino acid sequence of SEQ ID NO:21,
   CDR1 includes an amino acid sequence of SEQ ID NO: 13 or SEQ ID NO: 14,
   CDR2 includes an amino acid sequence of SEQ ID NO: 15 or SEQ ID NO: 16,
   CDR3 includes an amino acid sequence of SEQ ID NO: 17 or SEQ ID NO: 18, and
   the framework region 3 includes an amino acid sequence of SEQ ID NO: 19.
18. The method for producing a fusion protein according to 13 or 14 described above, in which in the variable region of the heavy chain, the antibody includes an amino acid sequence obtained by modifying an amino acid sequence of SEQ ID NO:21 with substitution, deletion, or addition of 1 to 5 amino acids,
   CDR1 includes an amino acid sequence of SEQ ID NO: 13 or SEQ ID NO: 14,
   CDR2 includes an amino acid sequence of SEQ ID NO: 15 or SEQ ID NO: 16,
   CDR3 includes an amino acid sequence of SEQ ID NO: 17 or SEQ ID NO: 18, and
   the framework region 3 includes an amino acid sequence of SEQ ID NO: 19.
19. The method for producing a fusion protein according to 13 or 14 described above, in which in the variable region of the heavy chain, the antibody includes an amino acid sequence obtained by modifying an amino acid sequence of SEQ ID NO:21 with substitution, deletion, or addition of 1 to 3 amino acids,
   CDR1 includes an amino acid sequence of SEQ ID NO: 13 or SEQ ID NO: 14,
   CDR2 includes an amino acid sequence of SEQ ID NO: 15 or SEQ ID NO: 16,
   CDR3 includes an amino acid sequence of SEQ ID NO: 17 or SEQ ID NO: 18, and
   the framework region 3 includes an amino acid sequence of SEQ ID NO: 19.
20. The method for producing a fusion protein according to any one of 13 to 19 described above, in which the variable region of the light chain in the antibody includes an amino acid sequence of SEQ ID NO:20.
21. The method for producing a fusion protein according to any one of 13 to 19 described above, in which in the variable region of the light chain, the antibody includes an amino acid sequence having 80% or more of identity with an amino acid sequence of SEQ ID NO:20,
   CDR1 includes an amino acid sequence of SEQ ID NO:8 or SEQ ID NOV,
   CDR2 includes an amino acid sequence of SEQ ID NO: 10 or SEQ ID NO: 11, or an amino acid sequence Lys-Val-Ser, and
   CDR3 includes an amino acid sequence of SEQ ID NO: 12.
22. The method for producing a fusion protein according to any one of 13 to 19 described above, in which in the variable region of the light chain, the antibody includes an amino acid sequence having 90% or more of identity with an amino acid sequence of SEQ ID NO:20,
   CDR1 includes an amino acid sequence of SEQ ID NO:8 or SEQ ID NOV,
   CDR2 includes an amino acid sequence of SEQ ID NO: 10 or SEQ ID NO: 11, or an amino acid sequence Lys-Val-Ser, and
   CDR3 includes an amino acid sequence of SEQ ID NO: 12.
23. The method for producing a fusion protein according to any one of 13 to 19 described above, in which in the variable region of the light chain, the antibody includes an amino acid sequence obtained by modifying an amino acid sequence of SEQ ID NO:20 with substitution, deletion, or addition of 1 to 5 amino acids,
   CDR1 includes an amino acid sequence of SEQ ID NO:8 or SEQ ID NOV,
   CDR2 includes an amino acid sequence of SEQ ID NO: 10 or SEQ ID NO: 11, or an amino acid sequence Lys-Val-Ser, and
   CDR3 includes an amino acid sequence of SEQ ID NO: 12.
24. The method for producing a fusion protein according to any one of 13 to 19 described above, in which in the variable region of the light chain, the antibody includes an amino acid sequence obtained by modifying an amino acid sequence of SEQ ID NO:20 with substitution, deletion, or addition of 1 to 3 amino acids,
   CDR1 includes an amino acid sequence of SEQ ID NO:8 or SEQ ID NOV,
   CDR2 includes an amino acid sequence of SEQ ID NO: 10 or SEQ ID NO: 11, or an amino acid sequence Lys-Val-Ser, and
   CDR3 includes an amino acid sequence of SEQ ID NO: 12.
25. The method for producing a fusion protein according to 15 described above, in which the heavy chain of the antibody includes an amino acid sequence of SEQ ID NO:23.
26. The method for producing a fusion protein according to 20 or 25 described above, in which the light chain of the antibody includes an amino acid sequence of SEQ ID NO:22.
27. The method for producing a fusion protein according to any one of 1 to 26 described above, in which the antibody is Fab, F(ab')₂, or F(ab').
28. The method for producing a fusion protein according to any one of 1 to 27 described above, in which the human lysosomal enzyme in the fusion protein is bound to a C-terminal side or an N-terminal side of the light chain of the antibody.
29. The method for producing a fusion protein according to 28 described above, in which the human lysosomal enzyme in the fusion protein is bound to the C-terminal side or the N-terminal side of the light chain directly or via a linker.
30. The method for producing a fusion protein according to 28 described above, in which the human lysosomal enzyme in the fusion protein is bound to a C-terminal side or an N-terminal side of the heavy chain via a linker.
31. The method for producing a fusion protein according to 29 or 30 described above, in which a linker sequence is peptide including 1 to 50 amino acid residues.
32. The method for producing a fusion protein according to 31 described above, in which the linker is peptide including an amino acid sequence selected from the group consisting of single glycine, single serine, an amino acid sequence Gly-Ser, an amino acid sequence Gly-Gly-Ser, an amino acid sequence of SEQ ID NO:1, an amino acid sequence of SEQ ID NO:2, an amino acid sequence of SEQ ID NO:3, an amino acid sequence of SEQ ID NO:4, and an amino acid sequence including 1 to 10 consecutive amino acid sequences.
33. The method for producing a fusion protein according to any one of 1 to 32 described above, in which the human lysosomal enzyme in the fusion protein is human α-L-iduronidase.
34. The method for producing a fusion protein according to 33 described above, in which the antibody in the fusion protein is Fab,
   the light chain of the antibody includes an amino acid sequence of SEQ ID NO:22, and
   the heavy chain of the antibody is bound to the human α-L-iduronidase via a linker including an amino acid sequence of SEQ ID NO:4 on the C-terminal side such that the fusion protein forms an amino acid sequence of SEQ ID NO:27.
35. The method for producing a fusion protein according to 33 described above, in which the antibody in the fusion protein is Fab,
   the light chain of the antibody includes an amino acid sequence of SEQ ID NO:22, and
   the heavy chain of the antibody includes an amino acid sequence of SEQ ID NO:23, and is bound to the human α-L-iduronidase including an amino acid sequence of SEQ ID NO:6 via a linker including an amino acid sequence of SEQ ID NO:4 on the C-terminal side.
36. The method for producing a fusion protein according to 33 described above, in which the human α-L-iduronidase includes an amino acid sequence of SEQ ID NO:6 or 7.

### Advantageous Effects of Invention

According to the present invention, it is possible to provide the fusion protein of the anti-transferrin receptor antibody and the lysosomal enzyme, which is purified to a purity at which the fusion protein can be clinically used as a therapeutic agent of a lysosomal disease accompanied with central nervous system disorders. In particular, it is possible to provide the fusion protein of the anti-transferrin receptor antibody and the human IDUA, which is purified to a purity at which the fusion protein can be clinically used as a therapeutic agent of Hunter syndrome accompanied with the central nervous system disorders.

### Brief Description of Drawings

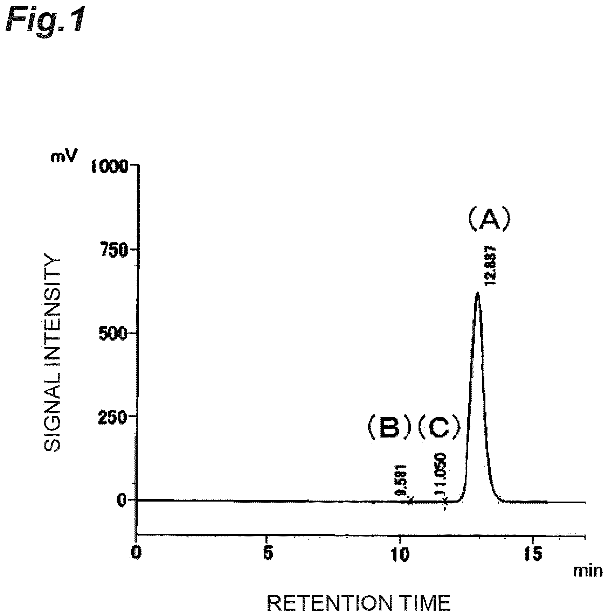

FIG. 1 is a diagram illustrating a SE-HPLC chart of a purified product of humanized anti-hTfR antibody-hIDUA obtained in Example 4, in which a vertical axis represents an absorbance at 215 nm, a horizontal axis represents a retention time (minutes), (A) represents a peak derived from a monomer of the humanized anti-hTfR antibody-hIDUA, and (B) and (C) each represent a peak derived from a polymer of the humanized anti-hTfR antibody-hIDUA.

### Description of Embodiments

The present invention relates to a method for producing a protein in which an anti-transferrin receptor antibody (an anti-TfR antibody) and a human lysosomal enzyme are bound. Here, the antibody bound to the lysosomal enzyme is not particularly limited to animal species of the antibody, insofar as the antibody has properties to be specifically bound to an antigen, and is particularly a human antibody or a humanized antibody. For example, the antibody may be an antibody of a non-human mammal, or may be a chimeric antibody of a human antibody and an antibody of a non-human mammal.

The human antibody referred to an antibody of which the entirety is encoded by a gene derived from human. However, in order to enhance an expression efficiency of the gene, an antibody encoded with a gene that is obtained by mutating an original human gene is also a human antibody. In addition, an antibody obtained by combining two or more genes encoding the human antibody and substituting a part of a certain human antibody with a part of another human antibody is also a human antibody. The human antibody includes three complementarity determining regions (CDR) of a light chain of immunoglobulin and three complementarity determining regions (CDR) of a heavy chain of the immunoglobulin. Three CDRs of the light chain of the immunoglobulin are referred to as CDR1, CDR2, and CDR3 in this order from an N-terminal side. Three CDRs of the heavy chain of the immunoglobulin are referred to as CDR1, CDR2, and CDR3 in this order from the N-terminal side. An antibody obtained by substituting CDR of a certain human antibody with CDR of another human antibody to modify the antigen specificity, the affinity, and the like of the human antibody is also a human antibody.

In the present invention, an antibody obtained by modifying the gene of the original human antibody and introducing a mutation such as substitution, deletion, or addition to an amino acid sequence of the original antibody is also referred to as a human antibody. In a case where an amino acid in the amino acid sequence of the original antibody is substituted with another amino acid, the number of amino acids to be substituted is preferably 1 to 20, more preferably 1 to 10, even more preferably 1 to 5, and much more preferably 1 to 3. In a case where an amino acid in the amino acid sequence of the original antibody is deleted, the number of amino acids to be deleted is preferably 1 to 20, more preferably 1 to 10, even more preferably 1 to 5, and much more preferably 1 to 3. In addition, an antibody obtained by introducing a combined mutation of the substitution and the deletion of amino acids is also a human antibody. In a case where an amino acid is added, preferably 1 to 20 amino acids, more preferably 1 to 10 amino acids, even more preferably 1 to 5 amino acids, and much more preferably 1 to 3 amino acids are added in the amino acid sequence, or on an N-terminal side or a C-terminal side of the original antibody. An antibody obtained by introducing a combined mutation of the addition, the substitution, and the deletion of amino acids is also a human antibody. The amino acid sequence of the antibody obtained by introducing the mutation exhibits preferably 80% or more of identity, more preferably 85% or more of identity, even more preferably 90% or more of identity, much more preferably 95% or more of identity, and still even more preferably 98% or more of identity, with the amino acid sequence of the original antibody. That is, in the present invention, the "gene derived from human" includes not only the original gene derived from human but also a gene obtained by modifying the original gene derived from human.

In the present invention, the term "humanized antibody" is referred to as an antibody in which an amino acid sequence of a part of a variable region (for example, in particular, the whole or a part of CDR) is derived from a non-human mammal, and the other region is derived from human. Examples of the humanized antibody include an antibody produced by substituting three complementarity determining regions (CDR) of the light chain of the immunoglobulin configuring the human antibody and three complementarity determining regions (CDR) of the heavy chain of the immunoglobulin with CDR of other mammals. Biological species of the other mammals from which CDR is derived, the CDR being grafted into a suitable position of the human antibody, are not particularly limited insofar as the biological species are a non-human mammal, but are preferably mice, rats, rabbits, horses, or non-human primates, more preferably mice and rats, and for example, mice.

In the present invention, a case where the antibody is the human antibody or the humanized antibody will be described below in detail. The light chain of the human antibody and the humanized antibody includes a λ chain and a κ chain. The light chain configuring the antibody may be either the λ chain or the κ chain. In addition, the heavy chain of the human antibody and the humanized antibody includes a γ chain, a µ chain, a α chain, a σ chain, and a ε chain, which respectively correspond to IgG, IgM, IgA, IgD, and IgE. The heavy chain configuring the antibody may be any of the γ chain, the µ chain, the α chain, the σ chain, and the ε chain, but is preferably the γ chain. Further, the γ chain of the heavy chain of the antibody includes a γ1 chain, a γ2 chain, a γ3 chain, and a γ4 chain, which respectively correspond to IgG1, IgG2, IgG3, and IgG4. In a case where the heavy chain configuring the antibody is the γ chain, the γ chain may be any of the γ1 chain, the γ2 chain, the γ3 chain, and the γ4 chain, but is preferably the γ1 chain or the γ4 chain. In a case where the antibody is the humanized antibody or the human antibody and is IgG, the light chain of the antibody may be either the λ chain or the κ chain, and heavy chain of the antibody may be any of the γ1 chain, the γ2 chain, the γ3 chain, and the γ4 chain, but is preferably the γ1 chain or the γ4 chain. For example, a preferred embodiment of the antibody includes an antibody in which the light chain is the κ chain and the heavy chain is the γ1 chain, and an antibody in which the light chain is the λ chain and the heavy chain is the γ1 chain.

In the present invention, the term "chimeric antibody" indicates an antibody obtained by connecting fragments of two or more different antibodies derived from two or more different species.

A chimeric antibody of a human antibody and another mammalian antibody is an antibody obtained by substituting a part of the human antibody with a part of the non-human mammalian antibody. The antibody includes a Fc region, a Fab region, and a hinge region described below. Specific examples of such a chimeric antibody include a chimeric antibody in which the Fc region is derived from the human antibody, whereas the Fab region is derived from another mammalian antibody. The hinge region is derived from either the human antibody or another mammalian antibody. In contrast, examples of the chimeric antibody include a chimeric antibody in which the Fc region is derived from another mammalian, whereas the Fab region is derived from the human antibody. The hinge region may be derived from either the human antibody or another mammalian antibody.

In addition, the antibody may include a variable region and a constant region. Another specific example of the chimeric antibody includes a chimeric antibody in which a constant region (C_{H}) of the heavy chain and a constant region (C_{L}) of the light chain are derived from the human antibody, whereas a variable region (V_{H}) of the heavy chain and a variable region (V_{L}) of the light chain are derived from another mammalian antibody, or a chimeric antibody in which the constant region (C_{H}) of the heavy chain and the constant region (C_{L}) of the light chain are derived from another mammalian antibody, whereas the variable region (V_{H}) of the heavy chain and the variable region (V_{L}) of the light chain are derived from the human antibody. Here, the biological species of another mammal are not particularly limited insofar as the biological species are a non-human mammal, but is preferably, mice, rats, rabbits, horses, or non-human primates, and more preferably mice.

A chimeric antibody of a human antibody and a mouse antibody, in particular, is referred to as a "human/mouse chimeric antibody". Examples of the human/mouse chimeric antibody include a chimeric antibody in which the Fc region is derived from the human antibody, whereas the Fab region is derived from the mouse antibody, or a chimeric antibody in which the Fc region is derived from the mouse antibody, whereas the Fab region is derived from the human antibody. The hinge region is derived from either the human antibody or the mouse antibody. Another specific example of the human/mouse chimeric antibody includes a chimeric antibody in which the constant region (C_{H}) of the heavy chain and the constant region (C_{L}) of the light chain are derived from the human antibody, whereas the variable region (V_{H}) of the heavy chain and the variable region (V_{L}) of the light chain are derived from the mouse antibody, or a chimeric antibody in which the constant region (C_{H}) of the heavy chain and the constant region (C_{L}) of the light chain are derived from the mouse antibody, whereas the variable region (V_{H}) of the heavy chain and the variable region (V_{L}) of the light chain are derived from the human antibody.

Originally, the antibody has a basic structure including a total of four polypeptide chains of two immunoglobulin light chains and two immunoglobulin heavy chains. However, in the present invention, the "antibody" includes not only the antibody having a basic structure, but also:
(1) an antibody including a total of two polypeptide chains of one immunoglobulin light chain and one immunoglobulin heavy chain; and as described below in detail,
(2) a single-chain antibody obtained by binding a linker sequence to the C-terminal side of the immunoglobulin light chain and further binding the immunoglobulin heavy chain to the C-terminal side;
(3) a single-chain antibody obtained by binding the linker sequence to the C-terminal side of the immunoglobulin heavy chain and further binding the immunoglobulin light chain to the C-terminal side; and
(4) an antibody including the Fab region in which the Fc region is removed from the basic structure of the antibody, in the original meaning, and an antibody including the Fab region and the whole or a part of the hinge region (including Fab, F(ab'), and F(ab')₂).

Here, Fab indicates a molecule in which one light chain including the variable region and the C_{L} region (the constant region of the light chain), and one heavy chain including the variable region and the C_{H}1 region (a region 1 of the constant region of the heavy chain) are bound by a disulfide bond between cysteine residues present on each of the chains. In Fab, the heavy chain may include a part of the hinge region in addition to the variable region and the C_{H}1 region (the region 1 of the constant region of the heavy chain), in such a case, the hinge region lacks the cysteine residue that is present on the hinge region to bind the heavy chains of the antibody. In Fab, the light chain and the heavy chain are bound by a disulfide bond formed between the cysteine residue present in the constant region (the C_{L} region) of the light chain and the cysteine residue present in the constant region (the C_{H}1 region) of the heavy chain or the hinge region. The heavy chain forming Fab is referred to as a Fab heavy chain. Since Fab lacks the cysteine residue that is present in the hinge region to bind the heavy chains of the antibody, Fab includes one light chain and one heavy chain. The light chain configuring Fab includes the variable region and the C_{L} region. The heavy chain configuring Fab may include the variable region and the C_{H}1 region, or may include a part of the hinge region in addition to the variable region and the C_{H}1 region. However, in this case, the hinge region is selected not to include the cysteine residue binding the heavy chains such that the disulfide bond is not formed between two heavy chains in the hinge region. In F(ab'), such a heavy chain includes the whole or a part of the hinge region including the cysteine residue binding the heavy chains, in addition to the variable region and the C_{H}1 region. F(ab')₂ indicates a molecule in which two F(ab')s are bound by a disulfide bond between the cysteine residues present in each hinge region. The heavy chain forming F(ab') or F(ab')₂ is referred to as a Fab' heavy chain. In addition, a polymer such as a dimer or a trimer obtained by binding a plurality of antibodies directly or via a linker is also an antibody. Further, the antibody is not limited thereto, and any one that includes a part of an immunoglobulin molecule and has the properties to be specifically bound to an antigen is also included in the "antibody" in the present invention. That is, in the present invention, the immunoglobulin light chain includes a chain that is derived from the immunoglobulin light chain and includes the amino acid sequence of the whole or a part of the variable region. In addition, the immunoglobulin heavy chain includes a chain that is derived from the immunoglobulin heavy chain and includes the amino acid sequence of the whole or a part of the variable region. Accordingly, for example, a chain in which the Fc region is removed is also the immunoglobulin heavy chain, insofar as the chain includes the amino acid sequence of the whole or a part of the variable region.

In addition, here, the Fc region or the Fc region indicates a region including a fragment including a C_{H}2 region (a region 2 of the constant region of the heavy chain) and a CH3 region (a region 3 of the constant region of the heavy chain), in the antibody molecule.

Further, in the present invention, the "antibody" also includes
(5) scFab, scF(ab'), and scF(ab')₂ in which the light chain and the heavy chain configuring Fab, F(ab'), or F(ab')₂ in (4) described above are bound via the linker sequence to be each single-chain antibody. Here, in scFab, scF(ab'), and scF(ab')₂, the linker sequence may be bound to the C-terminal side of the light chain, and then, the heavy chain may be further bound to the C-terminal side, and the linker sequence may be bound to the C-terminal side of the heavy chain, and then, the light chain may be further bound to the C-terminal side. Further, scFv in which the variable region of the light chain and the variable region of the heavy chain are bound via the linker sequence to be a single-chain antibody is also included in the antibody in the present invention. In scFv, the linker sequence may be bound to the C-terminal side of the variable region of the light chain, and then, the variable region of the heavy chain may be further bound to the C-terminal side, and the linker sequence may be bound to the C-terminal side of the variable region of the heavy chain, and then, the variable region of the light chain may be further bound to the C-terminal side.

Further, in this specification, the "antibody" also includes a full-length antibody, and any form of an antigen-binding fragment (an antibody fragment) in which a part of a full-length antibody is deficient, which is a broader concept including (4) and (5) described above, in addition to the antibodies in (1) to (5) described above.

The term "antigen-binding fragment" indicates a fragment of an antibody retaining at least a part of specific binding activity with an antigen. Examples of the binding fragment include Fab, Fab', F(ab')₂, the variable region (Fv), a single-chain antibody (scFv) in which the variable region (V_{H}) of the heavy chain and the variable region (V_{L}) of the light chain are connected by a suitable linker, a diabody that is a dimer of a polypeptide including the variable region (V_{H}) of the heavy chain and the variable region (V_{L}) of the light chain, a minibody that is a dimer of a molecule in which a part (C_{H}3) of the constant region is bound to the heavy chain (an H chain) of scFv, other low-molecular antibodies, and the like, in addition to the fragments in (4) and (5) described above. However, the binding fragment is not limited to such molecules insofar as the binding fragment has binding capability with an antigen. In addition, such binding fragments include not only a binding fragment obtained by treating a full-length molecule of an antibody protein with a suitable enzyme but also a protein produced in a suitable host cell by using a genetically modified antibody gene.

In the present invention, the "single-chain antibody" indicates a protein that is obtained by binding the linker sequence to the C-terminal side of the amino acid sequence including the whole or a part of the variable region of the immunoglobulin light chain, and further binding the amino acid sequence including the whole or a part of the variable region of the immunoglobulin heavy chain to the C-terminal side and can be specifically bound to a specific antigen. In addition, a protein that is obtained by binding the linker sequence to the C-terminal side of the amino acid sequence including the whole or a part of the variable region of the immunoglobulin heavy chain, and further binding the amino acid sequence including the whole or a part of the variable region of the immunoglobulin light chain to the C-terminal side and can be specifically bound to a specific antigen is also the "single-chain antibody" in the present invention. For example, the single-chain antibodies in (2) and (3) described above are included in the single-chain antibody. In the single-chain antibody in which the immunoglobulin light chain is bound to the C-terminal side of the immunoglobulin heavy chain via the linker sequence, in general, the Fc region lacks the immunoglobulin heavy chain. The variable region of the immunoglobulin light chain includes three complementarity determining regions (CDR) involved in the antigen specificity of the antibody. Similarly, the variable region of the immunoglobulin heavy chain also includes three CDRs. Such CDRs are a primary region determining the antigen specificity of the antibody. Accordingly, it is preferable that the single-chain antibody includes all of three CDRs of the immunoglobulin heavy chain and all of three CDRs of the immunoglobulin light chain. However, a single-chain antibody in which one or a plurality of CDRs are removed can also be used insofar as the antigen-specific affinity of the antibody is maintained.

In the single-chain antibody, the linker sequence disposed between the light chain and the heavy chain of the immunoglobulin is a peptide chain having preferably 2 to 50, more preferably 8 to 50, even more preferably 10 to 30, and much more preferably 12 to 18 or 15 to 25 amino acid residues, and for example, 15 or 25 amino acid residues. In such a linker sequence, there is no limitation on the amino acid sequence insofar as an anti-hTfR antibody of which both chains are connected by the amino acid sequence retains affinity for hTfR, and the amino acid sequence is preferably an amino acid sequence containing only glycine or glycine and serine, and examples thereof include an amino acid sequence Gly-Ser, an amino acid sequence Gly-Gly-Ser, an amino acid sequence Gly-Gly-Gly, an amino acid sequence Gly-Gly-Gly-Gly-Ser (SEQ ID NO:1), an amino acid sequence Gly-Gly-Gly-Gly-Gly-Ser (SEQ ID NO:2), an amino acid sequence Ser-Gly-Gly-Gly-Gly-Gly (SEQ ID NO:3), or a sequence in which the aforementioned amino acid sequences are repeated 2 to 10 times or 2 to 5 times. For example, in the case of obtaining ScFV by binding the variable region of the immunoglobulin light chain to the C-terminal side of the amino acid sequence including the entire region of the variable region of the immunoglobulin heavy chain via the linker sequence, a linker sequence (SEQ ID NO:4) including a total of 15 amino acids corresponding to three consecutive amino acid sequences Gly-Gly-Gly-Gly-Ser (SEQ ID NO:1) is preferably used.

In the present invention, the antigen specifically recognized by the antibody, for example, is a molecule (a surface antigen) present on the surface of vascular endothelial cells. Examples of such a surface antigen include an organic anion transporter such as a transferrin receptor (TfR), an insulin receptor, a leptin receptor, a lipoprotein receptor, an IGF receptor, and OATP-F, a monocarboxylic acid transporter such as MCT-8, and a Fc receptor, but the surface antigen is not limited thereto. The antigen is preferably a molecule (a surface antigen) present on the surface of human vascular endothelial cells.

Among the surface antigens described above, the organic anion transporter such as the transferrin receptor (TfR), the insulin receptor, the leptin receptor, the lipoprotein receptor, the IGF receptor, and OATP-F, and the monocarboxylic acid transporter such as MCT-8 are present on the surface of cerebral vascular endothelial cells forming a blood brain barrier. The antibody capable of recognizing such antigens can be bound to the cerebral vascular endothelial cells via the antigen. Then, the antibody bound to the cerebral vascular endothelial cells is capable of passing through the blood brain barrier and reaching the central nervous system. Accordingly, by binding a protein of interest to such an antibody, the protein of interest is capable of reaching the central nervous system. Examples of the protein of interest include a protein having a function to exert a drug effect in the central nervous system. Examples of the protein of interest include a lysosomal enzyme that is deficient or dysfunctional in lysosomal disease patients accompanied with central nervous system disorders. Such a lysosomal enzyme is not capable of reaching the central nervous system as it is, and does not exhibit the drug effect with respect to the central nervous system disorders of the patient, but by binding the lysosomal enzyme to the antibody, the lysosomal enzyme is capable of passing through the blood brain barrier, thereby enabling the central nervous system disorders found in the lysosomal disease patients to be improved.

In the present invention, the term "human transferrin receptor" or "hTfR" indicates a membrane protein including an amino acid sequence represented in SEQ ID NO:5. In one embodiment, the anti-hTfR antibody of the present invention is specifically bound to a region (the extracellular region of hTfR) from the 89th cysteine residue from the N-terminal side in the amino acid sequence represented by SEQ ID NO:5 to phenyl alanine of the C-terminal, but is not limited thereto.

A method for producing an antibody will be described below with an antibody against hTfR as an example. As the method for producing an antibody against hTfR, a method for producing an antibody by producing a recombinant human transferrin receptor (rhTfR) using cells to which an expression vector incorporating a hTfR gene is introduced, and immunizing animals such as mice with rhTfR is generally used. By collecting cells for producing an antibody against hTfR from the immunized animals, and fusing the cells with myeloma cells, it is possible to produce a hybridoma cell having capability to produce an antibody against hTfR.

In addition, it is also possible to acquire the cells for producing an antibody against hTfR by immunizing immunocompetent cells obtained from animals such as mice with rhTfR, in accordance with vitro immunization. In a case where the cells are immunized by the vitro immunization, there is no particular limitation on the animal species from which the immunocompetent cells are derived, but the species is preferably mice, rats, rabbits, guinea pigs, dogs, cats, horses, and primates including human, more preferably mice, rats, and human, and even more preferably mice and human. As mouse immunocompetent cells, for example, spleen cells prepared from mouse spleen can be used. As human immunocompetent cells, cells prepared from human peripheral blood, bone marrow, spleen, and the like can be used. In a case where the human immunocompetent cells are immunized by the vitro immunization, it is possible to obtain a human antibody against hTfR.

In the present invention, there is no particular limitation on the human lysosomal enzyme to be bound to the antibody, and examples thereof include lysosomal enzymes such as α-L-iduronidase, iduronate-2-sulfatase, glucocerebrosidase, β-galactosidase, a GM2 activator protein, β-hexosaminidase A, β-hexosaminidase B, N-acetyl glucosamine-1-phosphotransferase, α-mannosidase, β-mannosidase, galactosyl ceramidase, saposin C, aryl sulfatase A, α-L-fucosidase, aspartyl glucosaminidase, α-N-acetyl galactosaminidase, acidic sphingomyelinase, α-galactosidase A, β-glucuronidase, heparan N-sulfatase, α-N-acetyl glucosaminidase, acetyl CoAα-glucosaminide N-acetyl transferase, N-acetyl glucosamine-6-sulfate sulfatase, acidic ceramidase, amylo-1,6-glucosidase, sialidase, aspartyl glucosaminidase, palmitoyl protein thioesterase-1 (PPT-1), tripeptidyl peptidase-1 (TPP-1), hyaluronidase-1, CLN1, CLN2, CLN3, CLN6, and CLN8.

In a case where the antibody specifically recognizes the molecule (the surface antigen) present on the surface of the vascular endothelial cells, each of human lysosomal enzymes bound to the antibody can be used such that α-L-iduronidase can be used as a therapeutic agent for central nervous system disorders in Hunter syndrome, Hurler-Scheie syndrome, and Scheie syndrome, iduronate-2-sulfatase can be used as a therapeutic agent for central nervous system disorders in Hunter syndrome, glucocerebrosidase can be used as a therapeutic agent for central nervous system disorders in Gaucher's disease, β-galactosidase can be used as a therapeutic agent for central nervous system disorders in GM1-gangliosidosis Types 1 to 3, a GM2 activator protein can be used as a therapeutic agent for central nervous system disorders in GM2-gangliosidosis AB variant, β-hexosaminidase A can be used as therapeutic agent for central nervous system disorders in Sandhoffs disease and Tay-Sachs disease, β-hexosaminidase B can be used as a therapeutic agent for central nervous system disorders in Sandhoffs disease, N-acetyl glucosamine-1-phosphotransferase can be used as a therapeutic agent for central nervous system disorders in I-cell disease, α-mannosidase can be used as a therapeutic agent for central nervous system disorders in α-mannosidosis, β-mannosidase can be used as a therapeutic agent for central nervous system disorders in β-mannosidosis, galactosyl ceramidase can be used as a therapeutic agent for central nervous system disorders in Krabbe disease, saposin C can be used as a therapeutic agent for central nervous system disorders in Gaucher's disease-like storage disease, aryl sulfatase A can be used as a therapeutic agent for central nervous system disorders in metachromatic white matter degeneration (metachromatic leukodystrophy), α-L-fucosidase can be used as a therapeutic agent for central nervous system disorders in fucosidosis, aspartyl glucosaminidase can be used as a therapeutic agent for central nervous system disorders in aspartyl glucosaminuria, α-N-acetyl galactosaminidase can be used as a therapeutic agent for central nervous system disorders in Schindler disease and Kawasaki disease, acidic sphingomyelinase can be used as a therapeutic agent for central nervous system disorders in Niemann-Pick disease, α-galactosidase A can be used as a therapeutic agent for central nervous system disorders in Fabry disease, β-glucuronidase can be used as a therapeutic agent for central nervous system disorders in Sly syndrome, heparan N-sulfatase, α-N-acetyl glucosaminidase, acetyl CoAα-glucosaminide N-acetyl transferase, and N-acetyl glucosamine-6-sulfate sulfatase can be used as a therapeutic agent for central nervous system disorders in Sanfilippo syndrome, acidic ceramidase can be used as a therapeutic agent for central nervous system disorders in Farber disease, amylo-1,6-glucosidase can be used as a therapeutic agent for central nervous system disorders in Cori's disease (Forbes-Cori's disease), sialidase can be used as a therapeutic agent for central nervous system disorders in sialidase deficiency, aspartyl glucosaminidase can be used as a therapeutic agent for central nervous system disorders in aspartyl glucosaminuria, palmitoyl protein thioesterase-1 (PPT-1) can be used as a therapeutic agent for central nervous system disorders in neuronal ceroid lipofuscinosis or Santavuori-Haltia disease, tripeptidyl peptidase-1 (TPP-1) can be used as a therapeutic agent for central nervous system disorders in neuronal ceroid lipofuscinosis or Jansky-Bielschowsky disease, hyaluronidase-1 can be used as a therapeutic agent for central nervous system disorders in hyaluronidase deficiency, and CLN1, CLN2, CLN3, CLN6, and CLN8 can be used as a therapeutic agent for central nervous system disorders in Batten disease.

In a case where the antibody specifically recognizes the molecule (the surface antigen) present on the surface of the vascular endothelial cells, preferred examples of the lysosomal enzyme to be bound to the antibody may include human α-L-iduronidase (hIDUA). hIDUA is one type of lysosome enzymes having activity of hydrolyzing an iduronate bond present in glycosaminoglycan (GAG) molecules such as heparan sulfate or dermatan sulfate. Mucopolysaccharidosis I is a genetic disorder due to the mutation of a gene encoding the enzyme. The mucopolysaccharidosis I is classified into Hunter syndrome, Hurler-Scheie syndrome, and Scheie syndrome, in which the Hunter syndrome is severe, the Hurler-Scheie syndrome is intermediate, and the Scheie syndrome is mild. In patients of such syndrome, heparan sulfate and dermatan sulfate are accumulated in the tissue, resulting in various symptoms such as corneal opacity and mental development delay. However, in a case where the syndrome is mild, the mental development delay may not be observed. Since the fusion protein of the antibody and hIDUA is capable of degrading GAG accumulated in the brain tissue by passing through BBB, the fusion protein can be used as a therapeutic agent for central nervous system disorders by being administered to a patient with Hunter syndrome showing mental development delay.

In the present invention, the term "human α-L-iduronidase" or "hIDUA", in particular, indicates hIDUA including the same amino acid sequence as that of wild type hIDUA. The wild type hIDUA includes an amino acid sequence having 628 amino acids represented by SEQ ID NO:6. Variants of hIDUA including the amino acid sequence having 626 amino acids represented by SEQ ID NO:7 are also hIDUA. However, hIDUA is not limited thereto, and hIDUA in which the amino acid sequence of the wild type hIDUA is subjected to mutation such as substitution, deletion, and addition is also included in hIDUA, insofar as hIDUA has IDUA activity. In a case where the amino acid of the amino acid sequence of hIDUA is substituted with another amino acid, the number of amino acids to be substituted is preferably 1 to 10, more preferably 1 to 5, even more preferably 1 to 3, and much more preferably 1 to 2. In a case where the amino acid of the amino acid sequence of hIDUA is deleted, the number of amino acids to be deleted is preferably 1 to 10, more preferably 1 to 5, even more preferably 1 to 3, and much more preferably 1 to 2. In addition, a combined mutation of the substitution and the deletion of the amino acid can be performed. In a case where an amino acid is added to hIDUA, preferably 1 to 10, more preferably 1 to 5, even more preferably 1 to 3, and much more preferably 1 to 2 amino acids are added in the amino acid sequence of hIDUA, or on the N-terminal side or the C-terminal side. A combined mutation of the addition, the substitution, and the deletion of the amino acid can also be performed. The amino acid sequence of the mutated hIDUA has preferably 80% or more of identity, more preferably 85% or more of identity, even more preferably 90% or more of identity, much more preferably 95% or more of identity, and still even more preferably 99% or more of identity, with the amino acid sequence of the original hIDUA.

Note that, in the present invention, the identity between the amino acid sequence of the original protein (including the antibody) and the amino acid sequence of the mutated protein can be easily calculated by using a well-known homology calculator algorithm. Examples of such an algorithm include BLAST (Altschul SF. J Mol. Biol. 215. 403-10, (1990)), similarity search of Pearson and Lipman (Proc. Natl. Acad. Sci. USA. 85. 2444 (1988)), and a local homology algorithm of Smith and Waterman (Adv. Appl. Math. 2. 482-9 (1981)).

In addition, the substitution of the amino acid in the amino acid sequence of the original protein (including the antibody) with another amino acid, for example, occurs in an amino acid family including amino acids relevant each other to their side chains and chemical properties. Examples of such an amino acid family include the followings:
(1) an aspartic acid and a glutamic acid, which are an acidic amino acid;
(2) histidine, lysine, and arginine, which are a basic amino acid;
(3) phenyl alanine, tyrosine, and tryptophan, which are an aromatic amino acid;
(4) serine and threonine, which are a hydroxyamino acid;
(5) methionine, alanine, valine, leucine, and isoleucine, which are a hydrophobic amino acid;
(6) cysteine, serine, threonine, asparagine, and glutamine, which are a neutral hydrophilic amino acid;
(7) glycine and proline, which are an amino acid affecting the array of the peptide chain;
(8) asparagine and glutamine, which are an amide type amino acid;
(9) alanine, leucine, isoleucine, and valine, which are an aliphatic amino acid;
(10) alanine, glycine, serine, and threonine, which are an amino acid with small side chains;
(11) alanine and glycine, which are an amino acid with particularly small side chains; and
(12) valine, leucine, and isoleucine, which are an amino acid with branched chains.

In the present invention, hIDUA having IDUA activity indicates that the fusion protein obtained by fusing hIDUA and the antibody has 3% or more of activity with respect to the activity that the wild-type hIDUA intrinsically has. However, the activity is preferably 10% or more, more preferably 20% or more, even more preferably 50% or more, and much more preferably 80% or more, with respect to the activity that the wild-type hIDUA intrinsically has. The same applies to a case where hIDUA fused with the antibody is mutated. The antibody, for example, is an anti-hTfR antibody.

In the present invention, the "fusion protein" indicates a substance obtained by binding an antibody and a human lysosomal enzyme via a non-peptide linker or a peptide linker or directly. A method for binding the antibody and the human lysosomal enzyme will be described below in detail.

Examples of the method for binding the antibody and the human lysosomal enzyme include a method for binding the antibody and the human lysosomal enzyme via the non-peptide linker or the peptide linker. As the non-peptide linker, polyethylene glycol, polypropylene glycol, a copolymer of ethylene glycol and propylene glycol, polyoxyethylated polyol, polyvinyl alcohol, polysaccharides, dextran, polyvinyl ether, a biodegradable polymer, polymerized lipid, chitins, a hyaluronic acid, or derivatives thereof or a combination thereof can be used. The peptide linker is a peptide chain having 1 to 50 amino acids bound by a peptide bond or a derivative thereof, and whose N-terminal and C-terminal bind to either the antibody or the human lysosomal enzyme to let the antibody and the human lysosomal enzyme a covalently bind.

In a case where biotin-streptavidin is used as the non-peptide linker, the antibody may be bound to the biotin, the human lysosomal enzyme may be bound to the streptavidin, and the antibody and the human lysosomal enzyme may be bound via the bond between the biotin and the streptavidin, or on the contrary, the antibody may be bound to the streptavidin, the human lysosomal enzyme may be bound to the biotin, and the antibody and the human lysosomal enzyme may be bound via the bond between the biotin and the streptavidin.

One obtained by binding the antibody and the human lysosomal enzyme of the present invention using PEG as the non-peptide linker, in particular, is referred to as an antibody-PEG-human lysosomal enzyme. The antibody-PEG-human lysosomal enzyme can be produced by producing antibody-PEG by binding the antibody and PEG, and then, binding the antibody-PEG and the human lysosomal enzyme. Alternatively, the antibody-PEG-human lysosomal enzyme can be produced by binding the human lysosomal enzyme and PEG to produce human lysosomal enzyme-PEG, and then, binding the human lysosomal enzyme-PEG and the antibody. When binding PEG to the antibody and the human lysosomal enzyme, PEG modified with a functional group such as carbonate, carbonyl imidazole, active ester of a carboxylic acid, azlactone, cyclic imide thione, isocyanate, isothiocyanate, imidate, or aldehyde is used. Such a functional group introduced to PEG mainly reacts with an amino group in the antibody and the human lysosomal enzyme molecule to covalently bind PEG to the antibody and the human lysosomal enzyme. There is no particular limitation on the molecular weight and the shape of PEG to be used, but the average molecular weight (MW) is preferably MW = 300 to 60000, and more preferably MW = 500 to 20000. For example, PEG of which the average molecular weight is approximately 300, approximately 500, approximately 1000, approximately 2000, approximately 4000, approximately 10000, approximately 20000, and the like can be preferably used as the non-peptide linker.

For example, the antibody-PEG is obtained by mixing the antibody and polyethylene glycol (ALD-PEG-ALD) having an aldehyde group as a functional group such that a molar ratio of ALD-PEG-ALD to the antibody is 11, 12.5, 15, 110, 120, and the like, and adding a reducing agent such as NaCNBH₃ to cause a reaction. Next, by allowing the antibody-PEG to react with the human lysosomal enzyme in the presence of the reducing agent such as NaCNBH₃, the antibody-PEG-human lysosomal enzyme is obtained. On the contrary, it is also possible to obtain the antibody-PEG-human lysosomal enzyme by first binding the human lysosomal enzyme and ALD-PEG-ALD to produce the human lysosomal enzyme-PEG, and then, binding the human lysosomal enzyme-PEG and the antibody.

The antibody and the human lysosomal enzyme can also be bound by forming a peptide bond between the N-terminal or the C-terminal of the human lysosomal enzyme and the C-terminal side or the N-terminal side of the heavy chain or the light chain of the antibody via the linker sequence or directly. As described above, the fusion protein obtained by binding the antibody and the human lysosomal enzyme can be obtained by incorporating a DNA fragment in which cDNA encoding the human lysosomal enzyme is disposed in-frame into an expression vector for Eukaryote such as a mammalian cell and a yeast, on a 3'-terminal side or a 5'-terminal side of cDNA encoding the heavy chain or the light chain of the antibody directly or via a DNA fragment encoding the linker sequence, and culturing mammalian cells to which the expression vector is introduced. In the mammalian cells, in a case where the DNA fragment encoding the human lysosomal enzyme is bound to the heavy chain, an expression vector for mammalian cells incorporating a cDNA fragment encoding the light chain of the antibody is also introduced to the same host cell, and in a case where the DNA fragment encoding the human lysosomal enzyme is bound to the light chain, an expression vector for mammalian cells incorporating a cDNA fragment encoding the heavy chain of the antibody is also introduced to the host cell. In a case where the antibody is the single-chain antibody, the fusion protein in which the antibody and the human lysosomal enzyme are bound can be obtained by incorporating a DNA fragment in which cDNA encoding a single-chain antibody is connected, on the 5'-terminal side or the 3'-terminal side, to cDNA encoding the human lysosomal enzyme, directly or via the DNA fragment encoding the linker sequence, into the expression vector for Eukaryote such as a mammalian cell and a yeast, and expressing it in such cells to which the expression vector is introduced.

In a fusion protein in which the human lysosomal enzyme is bound to the C-terminal side of the light chain of the antibody, the antibody includes the amino acid sequence including the whole or a part of the variable region of the light chain and the amino acid sequence including the whole or a part of the variable region of the heavy chain, and the human lysosomal enzyme is bound to the C-terminal side of the light chain of the antibody. Here, the light chain of the antibody and the human lysosomal enzyme may be bound directly, or may be bound via the linker.

In a fusion protein in which the human lysosomal enzyme is bound to the C-terminal side of the heavy chain of the antibody, the antibody includes the amino acid sequence including the whole or a part of the variable region of the light chain and the amino acid sequence including the whole or a part of variable region of the heavy chain, and the human lysosomal enzyme is bound to the C-terminal side of the heavy chain of the antibody. Here, the heavy chain of the antibody and the human lysosomal enzyme may be bound directly, or may be bound via the linker.

In a fusion protein in which the human lysosomal enzyme is bound to the N-terminal side of the light chain of the antibody, the antibody includes the amino acid sequence including the whole or a part of the variable region of the light chain and the amino acid sequence including the whole or a part of the variable region of the heavy chain, and the human lysosomal enzyme is bound to the N-terminal side of the light chain of the antibody. Here, the light chain of the antibody and the human lysosomal enzyme may be bound directly, or may be bound via the linker.

In a fusion protein in which the human lysosomal enzyme is bound to the N-terminal side of the heavy chain of the antibody, the antibody includes the amino acid sequence including the whole or a part of the variable region of the light chain and the amino acid sequence including the whole or a part of the variable region of the heavy chain, and the human lysosomal enzyme is bound to the N-terminal side of the heavy chain of the antibody. Here, the heavy chain of the antibody and the human lysosomal enzyme may be bound directly, or may be bound via the linker.

In a case where the linker sequence is disposed between the antibody and the human lysosomal enzyme, the sequence includes preferably 1 to 50, more preferably 1 to 17, even more preferably 1 to 10, and much more preferably 1 to 5 amino acids, and the number of amino acids configuring the linker sequence can be suitably adjusted to 1, 2, 3, 1 to 17, 1 to 10, 10 to 40, 20 to 34, 23 to 31, 25 to 29, and the like, in accordance with the human lysosomal enzyme to be bound to the antibody. In such a linker sequence, there is no limitation on the amino acid sequence, insofar as the antibody connected by the linker sequence is capable of retaining affinity for hTfR and the human lysosomal enzyme connected by the linker sequence is capable of exerting the physiological activity of the protein in a physiological condition, and the amino acid sequence is preferably an amino acid sequence containing glycine and serine, and examples thereof include an amino acid sequence containing a single amino acid of either glycine or serine, an amino acid sequence Gly-Ser, an amino acid sequence Gly-Gly-Ser, an amino acid sequence Gly-Gly-Gly-Gly-Ser (SEQ ID NO: 1), an amino acid sequence Gly-Gly-Gly-Gly-Gly-Ser (SEQ ID NO:2), an amino acid sequence Ser-Gly-Gly-Gly-Gly-Gly (SEQ ID NO:3), or a sequence containing 1 to 50 amino acids including 1 to 10 or 2 to 5 of the aforementioned amino acid sequences consecutively linked, a sequence containing 2 to 17, 2 to 10, 10 to 40, 20 to 34, 23 to 31, and 25 to 29 amino acids, and the like. For example, a linker sequence including the amino acid sequence Gly-Ser and a sequence (SEQ ID NO:4) containing 15 amino acids including three consecutive amino acid sequences Gly-Gly-Gly-Gly-Ser (SEQ ID NO:1) are preferably used as the linker sequence. The same applies to a case where the antibody is the single-chain antibody.

Note that, in the present invention, in a case where a plurality of linker sequences are included in one peptide chain, for convenience, each linker sequence is referred to as a first linker sequence and a second linker sequence in order from the N-terminal side.

As a preferred embodiment of the antibody in a case where the antibody is an anti-human transferrin receptor antibody, an antibody, in which
in the variable region of the light chain:
   (a) CDR1 includes an amino acid sequence represented by SEQ ID NO:8 or 9,
   (b) CDR2 includes an amino acid sequence represented by SEQ ID NO:10 or 11, and
   (c) CDR3 includes an amino acid sequence represented by SEQ ID NO:12;
in the variable region of the heavy chain:
   (d) CDR1 includes an amino acid sequence represented by SEQ ID NO:13 or 14,
   (e) CDR2 includes an amino acid sequence represented by SEQ ID NO:15 or 16, and
   (f) CDR3 includes an amino acid sequence represented by SEQ ID NO:17 or 18, can be exemplified. Here, the antibody is preferably a human antibody or a humanized antibody.

A combination of the amino acid sequences of each CDR represented by (a) to (f) described above may be any combination, and examples thereof include combinations shown in Table 1.

**[Table 1]**

| (Table 1) Example 1 of Combination of Amino Acid Sequences of CDR of Light Chain and Heavy Chain | | | | | | |
|---|---|---|---|---|---|---|
| | Light chain | | | Heavy chain | | |
| | CDR1 | CDR2 | CDR3 | CDR1 | CDR2 | CDR3 |
| SEQ ID NO | 8 | 10 | 12 | 13 | 15 | 17 |
| | 8 | 10 | 12 | 14 | 16 | 18 |
| | 9 | 11 | 12 | 13 | 15 | 17 |
| | 9 | 11 | 12 | 14 | 16 | 18 |

As a more preferred embodiment of the antibody in a case where the antibody is the anti-human transferrin receptor antibody,
(x) an antibody in which the variable region of the light chain includes an amino acid sequence represented by SEQ ID NO:20, and the variable region of the heavy chain includes an amino acid sequence represented by SEQ ID NO:21, can be exemplified. Here, the antibody is preferably a human antibody or a humanized antibody.

Here, the amino acid sequence of the variable region of the light chain, which is represented by SEQ ID NO:20, includes amino acid sequences represented by SEQ ID NO:8 and SEQ ID NO:9 as CDR1, includes amino acid sequences represented by SEQ ID NO: 10 and SEQ ID NO: 11 as CDR2, and includes an amino acid sequence represented by SEQ ID NO: 12 as CDR3. In addition, the amino acid sequence of the variable region of the heavy chain, which is represented by SEQ ID NO:21, includes amino acid sequences represented by SEQ ID NO: 13 and SEQ ID NO: 14 as CDR1, includes amino acid sequences represented by SEQ ID NO: 15 and SEQ ID NO: 16 as CDR2, and includes amino acid sequences represented by SEQ ID NO: 17 and SEQ ID NO: 18 as CDR3. Further, the amino acid sequence of the variable region of the heavy chain includes an amino acid sequence represented by SEQ ID NO: 19 as the framework region 3 of the heavy chain.

However, a preferred embodiment of the antibody in a case where the antibody is the humanized antibody and the anti-human transferrin receptor antibody is not limited to (x) described above. For example, an antibody in which the amino acid sequence of the variable region of the light chain has 80% or more of identity with the amino acid sequence of the variable region of the light chain in (x) described above, and the amino acid sequence of the variable region of the heavy chain has 80% or more of identity with the amino acid sequence of the variable region of the heavy chain in (x) described above can be used in the present invention, insofar as the antibody has affinity for hTfR.

Further, an antibody in which the amino acid sequence of the variable region of the light chain has 85% or more of identity with the amino acid sequence of the variable region of the light chain in (x) described above, and the amino acid sequence of the variable region of the heavy chain has 85% or more of identity with the amino acid sequence of the variable region of the heavy chain in (x) described above,
an antibody in which the amino acid sequence of the variable region of the light chain has 90% or more of identity with the amino acid sequence of the variable region of the light chain in (x) described above, and the amino acid sequence of the variable region of the heavy chain has 90% or more of identity with the amino acid sequence of the variable region of the heavy chain in (x) described above, and
an antibody in which the amino acid sequence of the variable region of the light chain has 95% or more of identity with the amino acid sequence of the variable region of the light chain in (x) described above, and the amino acid sequence of the variable region of the heavy chain has 95% or more of identity with the amino acid sequence of the variable region of the heavy chain in (x) described above can also be used in the present invention, insofar as the antibody has affinity for hTfR.

Examples of the antibody in which the amino acid sequence of the variable region of the light chain has identity with the amino acid sequence of the variable region of the light chain in (x) described above, and the amino acid sequence of the variable region of the heavy chain has identity with the amino acid sequence of the variable region of the heavy chain in (x) described above, include
(x-a) an antibody in which the variable region of the light chain includes an amino acid sequence having 80% or more of sequence identity with an amino acid sequence represented by SEQ ID NO:20, and includes an amino acid sequence of SEQ ID NO:8 or 9 as CDR1, an amino acid sequence of SEQ ID NO: 10 or 11 as CDR2, and an amino acid sequence of SEQ ID NO: 12 as CDR3, respectively, and the variable region of the heavy chain includes an amino acid sequence having 80% or more of sequence identity with an amino acid sequence represented by SEQ ID NO:21, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively,
(x-b) an antibody in which the variable region of the light chain includes an amino acid sequence having 85% or more of sequence identity with an amino acid sequence represented by SEQ ID NO:20, and includes an amino acid sequence of SEQ ID NO:8 or 9 as CDR1, an amino acid sequence of SEQ ID NO: 10 or 11 as CDR2, and an amino acid sequence of SEQ ID NO: 12 as CDR3, respectively, and the variable region of the heavy chain includes an amino acid sequence having 85% or more of sequence identity with an amino acid sequence represented by SEQ ID NO:21, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively,
(x-c) an antibody in which the variable region of the light chain includes an amino acid sequence having 90% or more of sequence identity with an amino acid sequence represented by SEQ ID NO:20, and includes an amino acid sequence of SEQ ID NO:8 or 9 as CDR1, an amino acid sequence of SEQ ID NO: 10 or 11 as CDR2, and an amino acid sequence of SEQ ID NO: 12 as CDR3, respectively, and the variable region of the heavy chain includes an amino acid sequence having 90% or more of sequence identity with an amino acid sequence represented by SEQ ID NO:21, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively, and
(x-d) an antibody in which the variable region of the light chain includes an amino acid sequence having 95% or more of sequence identity with an amino acid sequence represented by SEQ ID NO:20, and includes an amino acid sequence of SEQ ID NO:8 or 9 as CDR1, an amino acid sequence of SEQ ID NO: 10 or 11 as CDR2, and an amino acid sequence of SEQ ID NO: 12 as CDR3, respectively, and the variable region of the heavy chain includes an amino acid sequence having 95% or more of sequence identity with an amino acid sequence represented by SEQ ID NO:21, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively.

Further, as a preferred embodiment of the antibody in a case where the antibody is the anti-human transferrin receptor antibody,
an antibody in which in the amino acid sequence of the variable region of the light chain in (x) described above, 1 to 5 amino acids in the amino acid sequence configuring the above are subjected to substitution, deletion, or addition, and in the amino acid sequence of the variable region of the heavy chain in (x) described above, 1 to 5 amino acids in the amino acid sequence configuring the above are subjected to substitution, deletion, or addition,
an antibody in which in the amino acid sequence of the variable region of the light chain in (x) described above, 1 to 3 amino acids in the amino acid sequence configuring the above are subjected to substitution, deletion, or addition, and in the amino acid sequence of the variable region of the heavy chain in (x) described above, 1 to 3 amino acids in the amino acid sequence configuring the above are subjected to substitution, deletion, or addition, and
an antibody in which in the amino acid sequence of the variable region of the light chain in (x) described above, 1 or 2 amino acids in the amino acid sequence configuring the above are subjected to substitution, deletion, or addition, and in the amino acid sequence of the variable region of the heavy chain in (x) described above, 1 or 2 amino acids in the amino acid sequence configuring the above are subjected to substitution, deletion, or addition can be used in the present invention, insofar as the antibody has affinity for hTfR. Here, the antibody is preferably a human antibody or a humanized antibody.

Examples of the antibody in which in the amino acid sequence of the variable region of the light chain in (x) described above, the amino acids in the amino acid sequence configuring the above are subjected to the substitution, the deletion, or the addition, and in the amino acid sequence of the variable region of the heavy chain in (x) described above, the amino acids in the amino acid sequence configuring the above are subjected to the substitution, the deletion, or the addition, include
(x-e)
   an antibody in which in the amino acid sequence of the variable region of the light chain, 1 to 5 amino acids in the amino acid sequence configuring the above are subjected to substitution, deletion, or addition, and an amino acid sequence of SEQ ID NO:8 or 9 as CDR1, an amino acid sequence of SEQ ID NO: 10 or 11 as CDR2, and an amino acid sequence of SEQ ID NO: 12 as CDR3 are included, respectively, and in the amino acid sequence of the variable region of the heavy chain, 1 to 5 amino acids in the amino acid sequence configuring the above are subjected to substitution, deletion, or addition, and an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3 are included, respectively,
(x-f)
   an antibody in which in the amino acid sequence of the variable region of the light chain, 1 to 3 amino acids in the amino acid sequence configuring the above are subjected to substitution, deletion, or addition, and an amino acid sequence of SEQ ID NO:8 or 9 as CDR1, an amino acid sequence of SEQ ID NO: 10 or 11 as CDR2, and an amino acid sequence of SEQ ID NO: 12 as CDR3 are included, respectively, and in the amino acid sequence of the variable region of the heavy chain, 1 to 3 amino acids in the amino acid sequence configuring the above are subjected to substitution, deletion, or addition, and an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3 are included, respectively, and
(x-g)
   an antibody in which in the amino acid sequence of the variable region of the light chain, 1 or 2 amino acids in the amino acid sequence configuring the above are subjected to substitution, deletion, or addition, and an amino acid sequence of SEQ ID NO:8 or 9 as CDR1, an amino acid sequence of SEQ ID NO: 10 or 11 as CDR2, and an amino acid sequence of SEQ ID NO: 12 as CDR3 are included, respectively, and in the amino acid sequence of the variable region of the heavy chain, 1 or 2 amino acids in the amino acid sequence configuring the above are subjected to substitution, deletion, or addition, and an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3 are included, respectively.

In (x-a) to (x-g) described above, a combination of the amino acid sequences of each CDR may be any combination, and examples thereof include combinations shown in Table 2. The same applies to (y-a) to (y-g) described below.

**[Table 2]**

| (Table 2) Example 2 of Combination of Amino Acid Sequences of CDR of Light Chain and Heavy Chain | | | | | | |
|---|---|---|---|---|---|---|
| | Light chain | | | Heavy chain | | |
| | CDR1 | CDR2 | CDR3 | CDR1 | CDR2 | CDR3 |
| SEQ ID NO | 8 | 10 | 12 | 13 | 15 | 17 |
| | 8 | 10 | 12 | 14 | 16 | 18 |
| | 9 | 11 | 12 | 13 | 15 | 17 |
| | 9 | 11 | 12 | 14 | 16 | 18 |

In the present invention, as a preferred embodiment of a case where the antibody is Fab that is the humanized antibody and the anti-human transferrin receptor antibody,
(y) an antibody that is Fab, in which the light chain includes an amino acid sequence represented by SEQ ID NO:22, and the heavy chain includes an amino acid sequence represented by SEQ ID NO:23, can be exemplified. Here, the light chain includes an amino acid sequence represented by SEQ ID NO:20 as the variable region, and the heavy chain includes an amino acid sequence represented by SEQ ID NO:21 as the variable region. In the present invention, the heavy chain configuring Fab is referred to as a Fab heavy chain. That is, the heavy chain including the amino acid sequence represented by SEQ ID NO:23 is the Fab heavy chain.

The preferred embodiment of the antibody in a case where the antibody is the humanized antibody and the anti-human transferrin receptor antibody is not limited to (y) described above. For example, an antibody in which the amino acid sequence of the light chain has 80% or more of identity with the amino acid sequence of the light chain in (y) described above, and the amino acid sequence of the heavy chain has 80% or more of identity with the amino acid sequence of the heavy chain in (y) described above can be used in the present invention, insofar as the antibody has affinity for hTfR.

Further, an antibody in which the amino acid sequence of the light chain has 85% or more of identity with the amino acid sequence of the light chain in (y) described above, and the amino acid sequence of the heavy chain has 85% or more of identity with the amino acid sequence of the heavy chain in (y) described above,
an antibody in which the amino acid sequence of the light chain has 90% or more of identity with the amino acid sequence of the light chain in (y) described above, and the amino acid sequence of the heavy chain has 90% or more of identity with the amino acid sequence of the heavy chain in (y) described above, and
an antibody in which the amino acid sequence of the light chain has 95% or more of identity with the amino acid sequence of the light chain in (y) described above, and the amino acid sequence of the heavy chain has 95% or more of identity with the amino acid sequence of the heavy chain in (y) described above can also be used in the present invention, insofar as the antibody has affinity for hTfR.

Examples of the antibody in which the amino acid sequence of the light chain has identity with the amino acid sequence of the light chain in (y) described above, and the amino acid sequence of the heavy chain has identity with the amino acid sequence of the heavy chain in (y) described above, include
(y-a) an antibody in which the light chain includes an amino acid sequence having 80% or more of sequence identity with an amino acid sequence represented by SEQ ID NO:22, and includes an amino acid sequence of SEQ ID NO:8 or 9 as CDR1, an amino acid sequence of SEQ ID NO: 10 or 11 as CDR2, and an amino acid sequence of SEQ ID NO: 12 as CDR3, respectively, and the heavy chain includes an amino acid sequence having 80% or more of sequence identity with an amino acid sequence represented by SEQ ID NO:23, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively,
(y-b) an antibody in which the light chain includes an amino acid sequence having 85% or more of sequence identity with an amino acid sequence represented by SEQ ID NO:22, and includes an amino acid sequence of SEQ ID NO:8 or 9 as CDR1, an amino acid sequence of SEQ ID NO: 10 or 11 as CDR2, and an amino acid sequence of SEQ ID NO: 12 as CDR3, respectively, and the heavy chain includes an amino acid sequence 85% or more of sequence identity with an amino acid sequence represented by SEQ ID NO:23, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively,
(y-c) an antibody in which the light chain includes an amino acid sequence having 90% or more of sequence identity with an amino acid sequence represented by SEQ ID NO:22, and includes an amino acid sequence of SEQ ID NO: 8 or 9 as CDR1, an amino acid sequence of SEQ ID NO: 10 or 11 as CDR2, and an amino acid sequence of SEQ ID NO: 12 as CDR3, respectively, and the heavy chain includes an amino acid sequence having 90% or more of sequence identity with an amino acid sequence represented by SEQ ID NO:23, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively, and
(y-d) an antibody in which the light chain includes an amino acid sequence having 95% or more of sequence identity with an amino acid sequence represented by SEQ ID NO:22, and includes an amino acid sequence of SEQ ID NO:8 or 9 as CDR1, an amino acid sequence of SEQ ID NO: 10 or 11 as CDR2, and an amino acid sequence of SEQ ID NO: 12 as CDR3, respectively, and the heavy chain includes an amino acid sequence having 95% or more of sequence identity with an amino acid sequence represented by SEQ ID NO:23, and includes an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3, respectively.

Further, as the preferred embodiment of the antibody in a case where the antibody is Fab that is the humanized antibody and the anti-human transferrin receptor antibody,
an antibody in which in the amino acid sequence of the light chain in (y) described above, 1 to 5 amino acids in the amino acid sequence configuring the above are subjected to substitution, deletion, or addition, and in the amino acid sequence of the heavy chain in (y) described above, 1 to 5 amino acids in the amino acid sequence configuring the above are subjected to substitution, deletion, or addition,
an antibody in which in the amino acid sequence of the light chain in (y) described above, 1 to 3 amino acids in the amino acid sequence configuring the above are subjected to substitution, deletion, or addition, and in the amino acid sequence of the heavy chain in (y) described above, 1 to 3 amino acids in the amino acid sequence configuring the above are subjected to substitution, deletion, or addition, and
an antibody in which in the amino acid sequence of the light chain in (y) described above, 1 or 2 amino acids in the amino acid sequence configuring the above are subjected to substitution, deletion, or addition, and in the amino acid sequence of the heavy chain in (y) described above, 1 or 2 amino acids in the amino acid sequence configuring the above are subjected to substitution, deletion, or addition can also be used in the present invention, insofar as the antibody has affinity for hTfR.

Examples of the antibody in which in the amino acid sequence of the light chain in (y) described above, the amino acids in the amino acid sequence configuring the above are subjected to the substitution, the deletion, or the addition, and in the amino acid sequence of the heavy chain in (y) described above, the amino acids in the amino acid sequence configuring the above are subjected to the substitution, the deletion, or the addition, include
(y-e)
   an antibody in which in the amino acid sequence of the light chain, 1 to 5 amino acids in the amino acid sequence configuring the above are subjected to substitution, deletion, or addition, and an amino acid sequence of SEQ ID NO:8 or 9 as CDR1, an amino acid sequence of SEQ ID NO: 10 or 11 as CDR2, and an amino acid sequence of SEQ ID NO: 12 as CDR3 are included, respectively, and in the amino acid sequence of the heavy chain, 1 to 5 amino acids in the amino acid sequence configuring the above are subjected to substitution, deletion, or addition, and an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3 are included, respectively,
(y-f)
   an antibody in which in the amino acid sequence of the light chain, 1 to 3 amino acids in the amino acid sequence configuring the above are subjected to substitution, deletion, or addition, and an amino acid sequence of SEQ ID NO:8 or 9 as CDR1, an amino acid sequence of SEQ ID NO: 10 or 11 as CDR2, and an amino acid sequence of SEQ ID NO: 12 as CDR3 are included, respectively, and in the amino acid sequence of the heavy chain, 1 to 3 amino acids in the amino acid sequence configuring the above are subjected to substitution, deletion, or addition, and an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3 are included, respectively, and
(y-g)
   an antibody in which in the amino acid sequence of the light chain, 1 or 2 amino acids in the amino acid sequence configuring the above are subjected to substitution, deletion, or addition, and an amino acid sequence of SEQ ID NO:8 or 9 as CDR1, an amino acid sequence of SEQ ID NO: 10 or 11 as CDR2, and an amino acid sequence of SEQ ID NO: 12 as CDR3 are included, respectively, and in the amino acid sequence of the heavy chain, 1 or 2 amino acids in the amino acid sequence configuring the above are subjected to substitution, deletion, or addition, and an amino acid sequence of SEQ ID NO: 13 or 14 as CDR1, an amino acid sequence of SEQ ID NO: 15 or 16 as CDR2, and an amino acid sequence of SEQ ID NO: 17 or 18 as CDR3 are included, respectively.

Examples of the preferred embodiment of a fusion protein in a case where the antibody is a humanized anti-human transferrin receptor antibody, and the human lysosomal enzyme is human α-L-iduronidase (hIDUA) include the following;
a fusion protein comprising the light chain of the humanized anti-hTfR antibody including an amino acid sequence represented by SEQ ID NO:22, and human α-L-iduronidase represented by SEQ ID NO:6 bound to the C-terminal side of the Fab heavy chain of the humanized anti-hTfR antibody including an amino acid sequence represented by SEQ ID NO:23 and via a linker sequence represented by SEQ ID NO:4.

In addition, examples of the preferred embodiment of the fusion protein in a case where the antibody is the humanized anti-human transferrin receptor antibody, and the human lysosomal enzyme is the human α-L-iduronidase (hIDUA) include the following;
a fusion protein in which the light chain of the humanized anti-hTfR antibody includes an amino acid sequence represented by SEQ ID NO:22, and
the Fab heavy chain of the humanized anti-hTfR antibody is bound to human α-L-iduronidase represented by SEQ ID NO:6 via a linker including an amino acid sequence represented by SEQ ID NON on the C-terminal side, and the whole includes the amino acid sequence represented by SEQ ID NO:27.

In the present invention, the fusion protein of the antibody and the human lysosomal enzyme, for example, can be produced by culturing mammalian cells that are artificially manipulated to produce the fusion protein by expressing or strongly expressing a gene encoding the fusion protein. In this case, the gene to be strongly expressed in the mammalian cells producing the fusion protein is generally introduced to the mammalian cells by being transformed with an expression vector incorporating the gene. In addition, there is no particular limitation on the mammalian cells used in this case, but cells derived from human, mice, and Chinese hamsters are preferable, and CHO cells derived from the ovary cells of the Chinese hamsters are particularly preferable. In the present invention, the fusion protein, in particular, indicates a recombinant fusion protein that is secreted into a medium when culturing the mammalian cells producing such a fusion protein.

The fusion protein of the antibody and the human lysosomal enzyme can also be produced by producing each of the antibody and the human lysosomal enzyme, and then, binding the antibody and the human lysosomal enzyme by a non-peptide linker or a peptide linker. In this case, the antibody and the human lysosomal enzyme can be produced as a recombinant protein by culturing the mammalian cells that are artificially manipulated to produce the antibody and the human lysosomal enzyme by expressing or strongly expressing a gene encoding the antibody and the human lysosomal enzyme.

As the expression vector for the use to incorporate and express the gene encoding the fusion protein, the antibody, or the human lysosomal enzyme, any expression vector can be used without any particular limitation insofar as the expression vector is capable of expressing the gene when introduced into the mammalian cells. The gene incorporated in the expression vector is disposed downstream of a DNA sequence (a gene expression regulatory site) capable of regulating the frequency of gene transcription in the mammalian cells. Examples of the gene expression regulatory site that can be used in the present invention include a promoter derived from cytomegalovirus, an SV40 early promoter, a human elongation factor-1 alpha (EF-1α) promoter, and a human ubiquitin C promoter.

The mammalian cells to which such an expression vector is introduced express a desired protein incorporated in the expression vector, and the expression level is not the same but different in accordance with each of the cells. Accordingly, in order to efficiently produce the recombinant protein, a step of selecting a cell with a high expression level of the desired protein from the mammalian cells to which the expression vector is introduced. In order to perform such a selection step, a gene that serves as a selectable marker is incorporated in the expression vector.

The most common selectable marker is an enzyme (a drug resistance marker) degrading drugs such as puromycin and neomycin. The mammalian cells are killed in the presence of the drugs at a certain concentration or more. However, since the mammalian cells to which the expression vector is introduced are capable of degrading the drugs with the drug resistance marker incorporated in the expression vector to detoxify the drugs or weaken the toxicity, the mammalian cells are viable even in the presence of the drugs. In a case where the expression vector incorporating the drug resistance marker as a selectable marker is introduced to the mammalian cells, and the mammalian cells are continuously cultured in a selective medium containing a drug corresponding to the drug resistance marker while gradually increasing the concentration of the drug, cells capable of proliferating even in the presence of the drug at a higher concentration are obtained. In the cells selected as described above, in general, the expression level of the gene encoding the desired protein incorporated in the expression vector also increases together with the drug resistance marker, and as a result thereof, the cell at a high expression level of the desired protein is selected.

In addition, glutamine synthetase (GS) can also be used as the selectable marker. The glutamine synthetase is an enzyme synthesizing glutamine from a glutamic acid and ammonia. In a case where the mammalian cells are cultured in a selective medium that contains an inhibitor of the glutamine synthetase, for example, methionine sulfoximine (MSX), and does not contain glutamine, the cells are killed. However, in a case where the expression vector incorporating the glutamine synthetase is introduced to the mammalian cells as the selectable marker, since the expression level of the glutamine synthetase increases, the cells are capable of proliferating in the presence of MSX at a higher concentration. In a case where the cells are continuously cultured while gradually increasing the concentration of MSX, cells capable of proliferating even in the presence of MSX at a higher concentration are obtained. In the cells selected as described above, in general, the expression level of the gene encoding the desired protein incorporated in the expression vector also increases together with the glutamine synthetase, and as a result thereof, the cell at a high expression level of the desired protein is selected.

In addition, dihydrofolate reductase (DHFR) can also be used as the selectable marker. In a case where DHFR is used as the selectable marker, the mammalian cells to which the expression vector is introduced are cultured in a selective medium containing a DHFR inhibitor such as methotrexate and aminopterin. In a case where the cells are cultured while gradually increasing the concentration of the DHFR inhibitor, cells capable of proliferating even in the presence of the DHFR inhibitor at a higher concentration are obtained. In the cells selected as described above, in general, the expression level of the gene encoding the desired protein incorporated in the expression vector also increases together with DHFR, and as a result thereof, the cell at a high expression level of the desired protein is selected.

An expression vector is known in which a glutamine synthetase (GS) as a selectable marker is disposed downstream of a gene encoding a desired protein via an internal ribosome entry site (IRES) (International Publication WO 2012/063799 and International Publication WO 2013/161958). The expression vector described in such literatures can be particularly preferably used in a method for producing a fusion protein of the present invention.

For example, an expression vector for expressing a protein, which includes a gene expression regulatory site and a gene encoding the protein downstream thereof, an internal ribosome entry site downstream thereof, and a gene encoding the glutamine synthetase downstream thereof, and further includes a dihydrofolate reductase gene or a drug resistance gene downstream of the gene expression regulatory site or another gene expression regulatory site different from the gene expression regulatory site, can be preferably used in the method for producing a fusion protein of the present invention. In such an expression vector, a promoter derived from cytomegalovirus, an SV40 early promoter, a human elongation factor-1 alpha promoter (a hEF-1α promoter), and a human ubiquitin C promoter are preferably used as the gene expression regulatory site or another gene expression regulatory site, and the hEF-1α promoter is particularly preferable.

In addition, internal ribosome entry sites derived from 5' untranslated regions of viruses or genes selected from the group consisting of viruses of Picornaviridae, Picornaviridae Aphthovirus, a hepatitis A virus, a hepatitis C virus, a coronavirus, a bovine enterovirus, a Theiler's murine encephalomyelitis virus, a Coxsackie B virus, a human immunoglobulin heavy chain binding protein gene, a drosophila antennapedia gene, and a drosophila ultrabithorax gene are preferably used as the internal ribosome entry site, and the internal ribosome entry site derived from the 5' untranslated region of the mouse encephalomyocarditis virus is particularly preferable. In a case where the internal ribosome entry site derived from the 5' untranslated region of the mouse encephalomyocarditis virus is used, not only a wild type internal ribosome entry site, but also an internal ribosome entry site in which a part of a plurality of initiation codons included in the wild type internal ribosome entry site is disrupted can be preferably used. In addition, in such an expression vector, the drug resistance gene that is preferably used is preferably a puromycin or neomycin resistance gene, and more preferably a puromycin resistance gene.

In addition, for example, an expression vector for expressing a protein, which includes a hEF-1α promoter, a gene encoding the protein downstream thereof, an internal ribosome entry site derived from a 5' untranslated region of a mouse encephalomyocarditis virus downstream thereof, and a gene encoding the glutamine synthetase downstream thereof, and further includes another gene expression regulatory site and a dihydrofolate reductase gene downstream thereof, the internal ribosome entry site being an internal ribosome entry site in which a part of a plurality of initiation codons included in a wild type internal ribosome entry site is disrupted, can be preferably used in the method for producing a fusion protein of the present invention. Examples of such an expression vector include an expression vector described in WO 2013/161958.

In addition, for example, an expression vector for expressing a protein, which includes a hEF-1α promoter, a gene encoding the protein downstream thereof, an internal ribosome entry site derived from a 5' untranslated region of a mouse encephalomyocarditis virus downstream thereof, and a gene encoding the glutamine synthetase downstream thereof, and further includes another gene expression regulatory site and a drug resistance gene downstream thereof, the internal ribosome entry site being an internal ribosome entry site in which a part of a plurality of initiation codons included in a wild type internal ribosome entry site is disrupted, can be preferably used in the method for producing a fusion protein of the present invention. Examples of such an expression vector include pE-mIRES-GS-puro described in WO 2012/063799 and pE-mIRES-GS-mNeo described in WO 2013/161958.

In the present invention, the mammalian cells to which the expression vector incorporating the gene encoding the fusion protein, the antibody, or the human lysosomal enzyme is introduced are selectively cultured in the selective medium in order to select the cells at a high expression level of the fusion protein, the antibody, or the human lysosomal enzyme.

In the selective culture, in a case where DHFR is used as the selectable marker, the concentration of the DHFR inhibitor contained in the selective medium is increased in a stepwise manner. In a case where the DHFR inhibitor is methotrexate, the maximum concentration is preferably 0.25 to 5 µM, more preferably 0.5 to 1.5 µM, and even more preferably approximately 1.0 µM.

In a case where GS is used as the selectable marker, the concentration of a GS inhibitor contained in the selective medium is increased in a stepwise manner. In a case where the GS inhibitor is MSX, the maximum concentration is preferably 100 to 1000 µM, more preferably 200 to 500 µM, and even more preferably approximately 300 µM. In addition, in this case, a medium not containing glutamine is generally used as the selective medium.

In a case where an enzyme degrading puromycin is used as the selectable marker, the maximum concentration of the puromycin contained in the selective medium is preferably 3 to 30 µg/mL, more preferably 5 to 20 µg/mL, and even more preferably approximately 10 µg/mL.

In a case where an enzyme degrading neomycin is used as the selectable marker, the maximum concentration of G418 contained in the selective medium is preferably 0.1 mg to 2 mg/mL, more preferably 0.5 to 1.5 mg/mL, and even more preferably approximately 1 mg/mL.

In addition, as a medium for culturing the mammalian cells, in addition to the medium used in the selective culture and a medium (a recombinant protein-production medium) used to produce the fusion protein, the antibody, or the human lysosomal enzyme described below, any medium can be used without any particular limitation insofar as the medium is capable of culturing the mammalian cells to proliferate, and a serum-free medium is preferably used.

The cells at a high expression level of the fusion protein, the antibody, or the human lysosomal enzyme selected by the selective culture are used in the production thereof as producing cells. The production of the fusion protein, the antibody, or the human lysosomal enzyme is performed by culturing the producing cells in the recombinant protein-production medium. Such culture is referred to as a production culture.

In the present invention, as the serum-free medium used as the recombinant protein-production medium, for example, a medium containing 3 to 700 mg/L of an amino acid, 0.001 to 50 mg/L of vitamins, 0.3 to 10 g/L of monosaccharides, 0.1 to 10000 mg/L of an inorganic salt, 0.001 to 0.1 mg/L of a trace element, 0.1 to 50 mg/L of nucleoside, 0.001 to 10 mg/L of a fatty acid, 0.01 to 1 mg/L of biotin, 0.1 to 20 µg/L of hydrocortisone, 0.1 to 20 mg/L of insulin, 0.1 to 10 mg/L of vitamin B12, 0.01 to 1 mg/L of putrescine, 10 to 500 mg/L of sodium pyruvate, and a water-soluble iron compound is preferably used. As desired, thymidine, hypoxanthine, a conventional pH indicator, and antibiotics may be added to the medium.

In addition, as the serum-free medium used as the recombinant protein-production medium, a DMEM/F12 medium (a mixed medium of DMEM and F12) may be used as a basic medium, and each of the media is well known to a person skilled in the art. In addition, as the serum-free medium, a DMEM(HG)HAM modified (R5) medium containing sodium hydrogen carbonate, L-glutamine, D-glucose, insulin, sodium selenite, diaminobutane, hydrocortisone, ferric (II) sulfate, asparagine, an aspartic acid, serine, and polyvinyl alcohol may be used. Further, a commercially available serum-free medium, for example, a CD OptiCHO^{™} medium, a CHO-S-SFM II medium, or a CD CHO medium (manufactured by Thermo Fisher Scientific Inc.), an EX-CELL^{™} 302 medium or an EX-CELL^{™} 325-PF medium (manufactured by SAFC Biosciences Inc), and the like can also be used as the basic medium. For example, an EX-CELL^{™} Advanced CHO Fed-batch medium (manufactured by SAFC Biosciences Inc) that is a serum-free medium containing 16 µmol/L of thymidine, 100 µmol/L of hypoxanthine, and 4 mmol/L of L-alanyl-L-glutamine can be preferably used in the culture of fusion protein-producing cells. In addition, for example, a CD OptiCHO^{™} medium (manufactured by Thermo Fisher Scientific Inc.) that is a serum-free medium containing 16 µmol/L of thymidine, 100 µmol/L of hypoxanthine, and 10 mg/L of insulin can also be preferably used in the culture of the fusion protein-producing cells.

In the production culture of the cells producing the fusion protein, the antibody, or the human lysosomal enzyme, the production culture is started by adjusting the density of the producing cells in the recombinant protein-production medium to preferably 0.2 × 10⁵ to 5 × 10⁵ cells/mL, more preferably 1 × 10⁵ to 4 × 10⁵ cells/mL, and even more preferably approximately 3 × 10⁵ cells/mL.

The production culture is performed while observing a cell viability (%) over time, and is performed such that the cell viability during a production culture period is maintained at preferably 80% or more, and more preferably 85% or more.

In addition, during the production culture period, a culture temperature is maintained at preferably 33.5°C to 37.5°C, and a dissolved oxygen saturation level in the production medium is maintained at preferably 28 to 32%, and more preferably approximately 30%. Here, the dissolved oxygen saturation level indicates the dissolution amount of oxygen when the saturated dissolution amount of oxygen is set to 100%, in the same condition.

In addition, during the production culture period, the production medium is stirred with an impeller. In this case, the rotational speed of the impeller is adjusted to preferably 50 to 100 rotations/minute, and more preferably 60 to 110 rotations/minute, and for example, 90 rotations/minute, 100 rotations/minute, 110 rotations/minute, and the like, and the rotational speed is suitably changed in accordance with the shape of the impeller or the like.

An example of the preferable initial condition of a culture condition in the production culture is that the density of the producing cells in the recombinant protein-production medium when starting the production culture is 3 × 10⁵ cells/mL, the culture temperature of the production culture period is 34°C to 37°C, the dissolved oxygen saturation level in the production medium is 30%, and the medium is stirred with the impeller rotating at a speed of approximately 100 rotations/minute.

The medium is collected after the completion of the production culture, and the collected medium is subjected to centrifugation or membrane filtration to remove the cells and the like, and a culture supernatant can be obtained. A fusion protein of interest contained in the culture supernatant is purified by a step using various chromatographies. A purification process can be performed at a room temperature or in a low temperature environment, and is performed preferably in a low temperature environment, and particularly preferably at a temperature of 1°C to 10°C.

Hereinafter, one embodiment of a purification method of the fusion protein of the antibody and the human lysosomal enzyme contained in the culture supernatant will be described in detail. This embodiment can be particularly preferably used in the case of a fusion protein in which the antibody is a Fab of human IgG antibody , and the human lysosomal enzyme is hIDUA. For example, this embodiment is preferable as the purification method of a fusion protein in which the light chain of the humanized anti-hTfR antibody includes an amino acid sequence represented by SEQ ID NO:22, the Fab heavy chain of the humanized anti-hTfR antibody is bound to human α-L-iduronidase represented by SEQ ID NO:6 via a linker including an amino acid sequence represented by SEQ ID NO:4 on the C-terminal side, and the whole includes the amino acid sequence represented by SEQ ID NO:27.

A first step of the purification process is a column chromatography using a material to which a substance having affinity for the antibody is bound as a solid phase. There is no particular limitation on the substance having affinity for the antibody, which is used in this embodiment, and the substance is preferably a substance having affinity for Fab, and in particular, a substance having affinity for the CH₁ region of the Fab heavy chain. An antibody against the region can be used as the substance having affinity for the antibody. In a case where Fab is the human IgG antibody, an anti-human IgG-CHi antibody is preferably used. In a case where the substance having affinity for the antibody is an antibody, in the solid phase, said antibody is bound to a carrier. Here, there is no particular limitation on the carrier, and for example, is agarose. By loading the culture supernatant, the fusion protein contained in the culture supernatant is bound to a column, and the column is washed, and then, the fusion protein is eluted from the column. As described above, it is possible to remove most of the contaminants.

Since the culture supernatant has a large volume, it is preferable to concentrate the culture supernatant before being provided to the purification process, but such a concentration operation is not essential. In the first step of the purification process, the arginine is added to the culture supernatant or a concentrate thereof before loading the culture supernatant or the concentrate thereof to the column. In this case, the arginine is added such that the concentration of the arginine in a solution is preferably 0.1 M to 1.0 M, more preferably 0.25 M to 1.0 M, even more preferably 0.3 M to 1.0 M, and much more preferably 0.3 to 0.5 M, and for example, is 0.4 M. In addition, the solid phase is equilibrated in advance with a buffer solution containing the arginine, before loading the culture supernatant. The concentration of the arginine in the buffer solution used in this case is preferably 0.1 M to 1.0 M, more preferably 0.2 M to 0.8 M, even more preferably 0.2 M to 0.6 M, and much more preferably 0.3 to 0.5 M, and for example, is 0.4 M. In addition, there is no particular limitation on the buffer solution used in this case, but is preferably an MES buffer solution, and the pH thereof is preferably 6.0 to 7.0, and more preferably approximately 6.5.

After washing the solid phase to which the fusion protein is bound, the fusion protein is eluted with an acidic buffer solution not containing a salt to collect a fraction containing the fusion protein. The buffer solution used in this case is preferably a glycine buffer solution or an acetic acid buffer solution, and the pH thereof is preferably 3.2 to 3.8, and more preferably 3.5. The pH of the solution containing the collected fusion protein is promptly adjusted to close to the neutral.

A second step of the purification process is an anion exchange column chromatography. There is no particular limitation on an anion exchanger used in this case, but the anion exchanger is preferably a strong anion exchanger, in particular, an anion exchanger having selectivity based on electrostatic interaction, hydrophobic interaction, and hydrogen bond formation. A multimodal anion exchanger can be preferably used in the present invention, and for example, a strong anion exchanger having a N-benzyl-N-methyl ethanol amine group as a functional group is particularly preferably used. Capto adhere (manufactured by GE Healthcare Inc.) is one of the multimodal strong anion exchangers having a N-benzyl-N-methyl ethanol amine group.

A buffer solution is added to the solution collected in the first step of the purification process before being provided to the anion exchange column chromatography, such that the pH is adjusted to close to the neutral. There is no particular limitation on the buffer solution used in this case, and for example, is an MES buffer solution. In addition, in this case, the pH is adjusted to preferably 5.5 to 6.5, and more preferably 5.7 to 6.3, and for example, 6.0. In addition, the solid phase is equilibrated in advance with a buffer solution. There is no particular limitation on the buffer solution used in this case, but is preferably an MES buffer solution, and the pH thereof is preferably 5.5 to 6.5, and for example, is 6.0.

The collected fraction of the column chromatography in the first step of the purification process, in which the pH is adjusted as described above, is loaded to the anion exchange column equilibrated in advance as described above, a non-absorption fraction containing the fusion protein is collected, and then, the column is washed with a buffer solution. A washing liquid obtained in this case is also collected as the non-absorption fraction. The buffer solution used to wash the column is preferably an MES buffer solution, and the pH thereof is preferably 5.5 to 6.5, and for example, is 6.0.

A third step of the purification process is a cation exchange chromatography. There is no particular limitation on which cation exchange resin to use in the cation exchange chromatography, but a weak cation exchanger is preferable, and a weak cation exchanger having selectivity based on both of the hydrophobic interaction and the hydrogen bond formation is more preferable. For example, a weak cation exchanger having a phenyl group, an amide bond, and a carboxyl group and having selectivity based on both of the hydrophobic interaction and the hydrogen bond formation, such as Capto MMC (manufactured by GE Healthcare Inc.), can be used.

In the cation exchange chromatography, the column is equilibrated in advance with a buffer solution. The buffer solution used in this case is preferably an MES buffer solution, and the pH thereof is preferably 6.0 to 7.0, and more preferably approximately 6.5.

The fusion protein-containing fraction and the washing liquid collected in the second step of the purification process are loaded to the cation exchange column, and the column to which the fusion protein is bound is washed, and then, the fusion protein is eluted. The elution can be performed by a buffer solution in which the concentration of a salt is increased. The buffer solution used in this case is preferably an MES buffer solution, and the pH thereof is preferably 6.0 to 7.0, and more preferably approximately 6.5. The salt added to the buffer solution is preferably sodium chloride, and the concentration of the salt is preferably in a range of 0.5 to 1.5 M, more preferably in a range of 0.8 to 1.2 M, and even more preferably approximately 1 M.

The pH of the fusion protein-containing fraction of the eluate obtained in the third step is adjusted to preferably 5.5 to 6.1, and more preferably approximately 5.8.

A fourth step of the purification process is a size exclusion column chromatography. This step is a step for removing low molecular-weight impurities such as endotoxin, multimeric complexes or decomposition products of the fusion protein, and the like to substantially obtain a pure fusion protein.

In addition, one or two or more column chromatographies may be further introduced to the above purification method which includes the column chromatography using the material to which the substance having affinity for the antibody is bound as the solid phase, the anion exchange column chromatography, the cation exchange column chromatography, and the size exclusion column chromatography in this order. Such an additional column chromatography, for example, is a column chromatography using a solid phase having affinity for a phosphoric acid group, a column chromatography using a material to which a substance having affinity for the antibody is bound as a solid phase, a hydrophobic column chromatography, an anion exchange column chromatography, a cation exchange column chromatography, and a dye affinity column chromatography. It is preferable that the dye affinity column chromatography using a blue triazine dye as a ligand is introduced before the first step of the purification process, or between the first step and the second step of the purification process.

In the purification process of the fusion protein, a step of inactivating viruses that may be brought from the culture supernatant can also be added. Such a virus inactivation step may be implemented before the first step of the purification process, may be implemented between the respective steps of the purification process, or may be implemented after the completion of the purification process, and for example, may be implemented before the first step of the purification process or between the first step and the second step of the purification process.

The virus inactivation step is performed by adding a nonionic surfactant to the solution containing the fusion protein and stirring at 20°C to 60°C for 2 to 6 hours. Preferred examples of the nonionic surfactant used in this case may include polysorbate 20, 80, and a tri-n-butyl phosphoric acid, or a mixture thereof.

The virus inactivation step can also be performed by using a virus removal membrane. By allowing the solution containing a humanized anti-hTfR antibody-hIDUA to pass through the virus removal membrane with a pore size of 35 nm or 20 nm, it is possible to remove viruses contained in the solution.

A purified product of the fusion protein obtained by using the method for producing a fusion protein of the present invention has a purity to the extent that the purified product can be used as medical drugs as it is. In particular, in the case of a fusion protein in which the light chain of the humanized anti-hTfR antibody includes an amino acid sequence represented by SEQ ID NO:22, the Fab heavy chain of the humanized anti-hTfR antibody is bound to human α-L-iduronidase represented by SEQ ID NO:6 via a linker including an amino acid sequence represented by SEQ ID NO:4 on the C-terminal side, and the whole includes the amino acid sequence represented by SEQ ID NO:27 (the humanized anti-hTfR antibody-hIDUA), the fusion protein can be used as medical drugs as it is.

The concentration of a host cell-derived protein (HCP) contained in the purified product of the fusion protein obtained by using the method for producing a fusion protein of the present invention, in particular, the humanized anti-hTfR antibody-hIDUA, is less than 100 ppm, for example, is less than 60 ppm, and less than 40 ppm, and for example, is 1 to 50 ppm, and 1 to 40 ppm.

A ratio of a polymer to the entire fusion protein contained in the purified product of the fusion protein obtained by using the method for producing a fusion protein of the present invention, in particular in the purified humanized anti-hTfR antibody-hIDUA, is less than 0.5%, for example, is less than 0.2%, less than 0.1%, and less than 0.05%, and for example, is 0.01 to 0.1%, and 0.001 to 0.1%.

In a case where the purified product of the fusion protein, in particular of the humanized anti-hTfR antibody-hIDUA, obtained by using the method for producing a fusion protein of the present invention is provided as medical drugs, the purified product can be provided as an aqueous preparation or a lyophilized preparation containing a suitable excipient. In the case of the aqueous preparation, the aqueous preparation may be filled in a vial, or can also be provided as a prefilled-type preparation that is filled in advance in a syringe. In the case of the lyophilized preparation, the lyophilized preparation is used by being dissolved in an aqueous solution before use.

### Examples

Hereinafter, the present invention will be described below in more detail with reference to Examples, but the present invention is not intended to be limited to Examples.

### [Example 1] Construction of Expression Vector for Humanized Anti-hTfR Antibody-hIDUA Fusion Protein

An expression vector for a humanized anti-hTfR antibody-hIDUA fusion protein was constructed by using a gene encoding a light chain including an amino acid sequence represented by SEQ ID NO:22 and a Fab region of a heavy chain including an amino acid sequence represented by SEQ ID NO:23 as an antibody region.

### [Construction of pE-neo Vector and pE-hygr Vector]

A pEF/myc/nuc vector (manufactured by Invitrogen Corporation) was digested with KpnI and NcoI, and a region containing an EF-1 promoter and the first intron thereof was cut out and was blunt-ended with T4 DNA polymerase. Separately, pCI-neo (manufactured by Invitrogen Corporation) was digested with BglII and EcoRI, and a region containing an enhancer/promoter and intron of CMV was cut, and then, was blunt-ended with T4 DNA polymerase. The region containing the EF-1α promoter and the first intron thereof (which was blunt-ended) was inserted thereto to construct a pE-neo vector. The pE-neo vector was digested with SfiI and BstXI, and a region of approximately 1 kbp containing a neomycin resistance gene was cut out. A hygromycin gene was amplified by a PCR reaction using pcDNA 3.1/Hygro(+) (manufactured by Invitrogen Corporation) as a template and using a primer Hyg-Sfi5' (SEQ ID NO: 13) and a primer Hyg-BstX3' (SEQ ID NO:14). The amplified hygromycin gene was digested with SfiI and BstXI, was inserted to the pE-neo vector to construct a pE-hygr vector. Note that, the construction of the pE-neo vector and the pE-hygr vector was performed with reference to Patent Literature (Japanese Patent No. 6279466).

### [Construction of pE-IRES-GS-puro]

An expression vector pPGKIH (Miyahara M. et. al., J. Biol. Chem. 275, 613-618 (2000)) was digested with a restriction enzyme (XhoI and BamHI), and a DNA fragment including an internal ribosome entry site (IRES) derived from a mouse encephalomyocarditis virus (EMCV), and a polyadenylylation region (mPGKpA) of a hygromycin resistance gene (Hyg^{r} gene) and mouse phosphoglycerate kinase (mPGK) was cut out. The DNA fragment was inserted between XhoI and BamHI sites of pBluescript SK(-) (manufactured by Stratagene Corporation) to be pBSK (IRES-Hygr-mPGKpA).

A DNA fragment including a part of IRES of EMCV was amplified by PCR using pBSK (IRES-Hygr-mPGKpA) as a template and using a primer IRES5' (SEQ ID NO:29) and a primer IRES3' (SEQ ID NO:30). The DNA fragment was digested with a restriction enzyme (XhoI and HindIII), and was inserted between XhoI and HindIII sites of pBSK (IRES-Hygr-mPGKpA) to be pBSK (NotI-IRES-Hygr-mPGKpA). pBSK (NotI-IRES-Hygr-mPGKpA) was digested with a restriction enzyme (NotI and BamHI), and was inserted between NotI and BamHI sites of the pE-hygr vector to be plasmid pE-IRES-Hygr.

An expression vector pPGKIH was digested with EcoRI, and a DNA fragment including a base sequence including a promoter region (mPGKp) of mPGK was cut out. The DNA fragment was inserted to an EcoRI site of pBluescript SK(-) (manufactured by Stratagene Corporation) to be an mPGK promoter/pBS(-). A DNA fragment including an mPGK promoter region (mPGKp) was amplified by PCR using the mPGK promoter/pBS(-) as a template and using a primer mPGKP5' (SEQ ID NO:31) and a primer mPGKP3' (SEQ ID NO:32). The DNA fragment was digested with a restriction enzyme (BglII and EcoRI), and was inserted between BglII and EcoRI sites of pCI-neo (manufactured by Promega Corporation) to be pPGK-neo. pE-IRES-Hygr was digested with a restriction enzyme (NotI and BamHI), and a DNA fragment (IRES-Hygr) was cut out, and was inserted between NotI and BamHI sites of pPGK-neo to be pPGK-IRES-Hygr.

A DNA fragment containing a GS gene was amplified by PCR using cDNA prepared from CHO-K1 cells as a template and using a primer GS5' (SEQ ID NO:33) and a primer GS3' (SEQ ID NO:34). The DNA fragment was digested with a restriction enzyme (BalI and BamHI), and was inserted between BalI and BamHI sites of pPGK-IRES-Hygr to be pPGK-IRES-GS-ΔpolyA.

A DNA fragment containing a puromycin resistance gene (puro^{r} gene) was amplified by PCR using pCAGIPuro (Miyahara M. et. al., J. Biol. Chem. 275, 613-618 (2000)) as a template and using a primer puro5' (SEQ ID NO:35) and a primer puro3' (SEQ ID NO:36). The DNA fragment was inserted to pT7Blue T-Vector (manufactured by Novagen, Inc.) to be pT7-puro.

pT7-puro was digested with a restriction enzyme (AflII and BstXI), and was inserted between AflII and BstXI sites of the expression vector pE-neo to be pE-puro.

A DNA fragment including an SV40 late polyadenylylation region was amplified by PCR using pE-puro as a template and using a primer SV40polyA5' (SEQ ID NO:37) and a primer SV40polyA3' (SEQ ID NO:38). The DNA fragment was digested with a restriction enzyme (NotI and HpaI), and was inserted between NotI and HpaI sites of the expression vector pE-puro to be pE-puro(XhoI). pPGK-IRES-GS-ΔpolyA was digested with a restriction enzyme (NotI and XhoI), and a DNA fragment including a IRES-GS region was cut out, and was inserted between NotI and XhoI sites of the expression vector pE-puro(XhoI) to be pE-IRES-GS-puro. Note that, the construction of pE-IRES-GS-puro was performed with reference to Patent Literature (Japanese Patent No. 6279466).

### [Construction of pE-mIRES-GS-puro(ΔE)]

A region from IRES to GS of EMCV was amplified by PCR using the expression vector pE-IRES-GS-puro as a template and using a primer mIRES-GS5' (SEQ ID NO:39) and a primer mIRES-GS3' (SEQ ID NO:40), and a DNA fragment disrupted by mutating an initiation codon (ATG) positioned second from a 5' side of IRES of EMCV was amplified. A DNA fragment including the region from IRES to GS described above was amplified by PCR using the expression vector pE-IRES-GS-puro as a template and using the DNA fragment and the primer IRES 5' described above. The DNA fragment was digested with a restriction enzyme (NotI and PstI), and the cut-out DNA fragment was inserted between NotI and PstI sites of pBluescript SK(-) (manufactured by Stratagene Corporation) to be mIRES/pBlueScript SK(-).

The expression vector pE-IRES-GS-puro was digested with SphI, and an SV40 enhancer region was cut. The remaining DNA fragment was subjected to self-ligation to be pE-IRES-GS-puro(ΔE). mIRES/pBlueScript SK(-) was digested with NotI and PstI, and a region including modified IRES (mIRES) and a part of a GS gene was cut out. Separately, pE-IRES-GS-puro(ΔE) was digested with NotI and PstI, and the region including mIRES described above and a part of the GS gene was inserted to construct pE-mIRES-GS-puro(ΔE).

### [Construction of pEM-hygr(LC3) and pE-mIRES-GSp-Fab-IDUA]

A DNA fragment (CMVE-EF-1αp-IFNβMAR) including a β-Globin matrix attachment region (MAR), a CMV enhancer, a human EF-1α promoter, Mini and BamHI cutting sites, and interferon β Mar was artificially synthesized (SEQ ID NO:41). A HindIII sequence was introduced to a 5' side of the DNA fragment, and an EcoRI sequence was introduced to a 3' side. The DNA fragment was digested with HindIII and EcoRI, and was inserted between HindIII and EcoRI sites of a pUC57 vector to be JCR69 in pUC57. A DNA fragment (IRES-HygroR-mPGKpA) including MluI and BamHI cutting sites, IRES, a hygromycin resistance gene, and an mPGK polyadenylation signal was artificially synthesized (SEQ ID NO:42). The DNA fragment was inserted between MluI and BamHI sites of JCR69 in pUC57 to be pEM hygro.

ADNAfragment (SEQ ID NO:26) including a gene encoding the full length of a light chain of a humanized anti-hTfR antibody including an amino acid sequence represented by SEQ ID NO:22 was artificially synthesized, and was inserted to pUC57-Amp to be JCR131 in pUC57-Amp. A MluI sequence was introduced to a 5' side of the DNA fragment, and a NotI sequence was introduced to a 3' side. The plasmid DNA was digested with MluI and NotI, and was incorporated between MluI-NotI of the expression vector pEM hygro. The obtained vector was pEM-hygr(LC3) that is an expression vector of the light chain of the humanized anti-hTfR antibody.

A DNA fragment including a base sequence represented by SEQ ID NO:28 including a gene encoding a protein including an amino acid sequence represented by SEQ ID NO:27 as a whole, in which human IDUA including an amino acid sequence represented by SEQ ID NO:6 was bound to a C-terminal side of the Fab region of the heavy chain of the humanized anti-hTfR antibody including an amino acid sequence represented by SEQ ID NO:27 via a linker sequence represented by SEQ ID NO:4, was artificially synthesized. A MluI sequence was introduced to a 5' side of the DNA fragment, and a NotI sequence was introduced to a 3' side. The DNA fragment was digested with MluI and NotI, and was incorporated between MluI and NotI of pE-mIRES-GS-puro(ΔE). The obtained vector was pE-mIRES-GSp-Fab-IDUA that is the expression vector of the protein in which hIDUA is bound to the C-terminal side of the Fab heavy chain of the humanized anti-hTfR antibody.

### [Example 2] Production of High Expression Cell Line of Humanized Anti-hTfR Antibody-hIDUA Fusion Protein

CHO cells (CHO-K1: obtained from American Type Culture Collection) were transformed with pEM-hygr(LC3) and pE-mIRES-GSp-Fab-IDUA constructed in Example 1 by the following method using NEPA21 (manufactured by Nepa Gene Co., Ltd.).

The transformation of the cells was generally performed by the following method. The CHO-K1 cells were suspended in a 1:1 mixed liquid of a CD OptiCHO^{™} medium (manufactured by Thermo Fisher Scientific Inc.) and PBS to a density of 2 × 10⁷ cells/mL. 50 µL of a cell suspension was sampled, and 50 µL of a pEM-hygr(LC3) plasmid DNA solution diluted to 200 µg/mL with a 1:1 mixed liquid of a CD OptiCHO^{™} medium and PBS was added thereto. Electroporation was implemented by using NEPA21 (manufactured by Nepa Gene Co., Ltd.) such that pEM-hygr(LC3) plasmid DNA was introduced to the cells. After overnight culture in the condition of 37°C and 5% CO₂, the cells were selectively cultured with a CD OptiCHO^{™} medium to which 0.5 mg/mL of hygromycin was added. pE-mIRES-GSp-Fab-IDUA plasmid DNA (digested with AhdI and linearized) was introduced to the obtained cells by the same method. After overnight culture in the condition of 37°C and 5% CO₂, the cells were selectively cultured with a CD OptiCHO^{™} medium to which 0.5 mg/mL of hygromycin and 10 µg/mL of puromycin were added. In the selective culture, the concentration of MSX was increased in a stepwise manner such that the concentration of MSX was finally 300 µM, and cells showing drug resistance selectively proliferated.

Next, the cells selected by the selective culture were seeded on a 96-well plate by limiting dilution such that one or less cells per 1 well proliferated, and were cultured for approximately 2 weeks such that each of the cells formed a monoclonal colony. Culture supernatants of the wells in which the monoclonal colony was formed were sampled, the content of the humanized antibody was determined by an ELISA method, and a high expression cell line of a humanized anti-hTfR antibody-hIDUA fusion protein was selected.

In this case, the ELISA method was generally implemented by the following method. 100 µL of a chicken anti-IDUA polyclonal antibody solution diluted to 5 µg/mL with 0.05 M hydrogencarbonate buffer solution was added to each well of 96-well microtiter plate (manufactured by Nunc Inc.), and was left to stand at a room temperature or 4°C for at least 1 hour such that the antibody was adsorbed on the plate. Next, each well was washed three times with tris-buffered saline (pH 8.0) to which 0.05% Tween20 was added (TBS-T), 300 µL of tris-buffered saline (pH 8.0) to which 1% BSA was added to each well, and the plate was left to stand at a room temperature for 1 hour. Next, each well was washed three times with TBS-T, and then, 100 µL of a culture supernatant or a purified product of the humanized anti-hTfR antibody-hIDUA fusion protein diluted to a suitable concentration with tris-buffered saline (pH 8.0) to which 0.1% BSA and 0.05% Tween20 were added (TBS-BT) was added to each well, and the plate was left to stand at a room temperature for 1 hour. Next, the plate was washed three times with TBS-T, and then, 50 µL of a HRP-labeled anti-human IgG polyclonal antibody solution diluted with TBS-BT was added to each well, and the plate was left to stand at a room temperature for 1 hour. Each well was washed three times with TBS-T, and then, color development was performed by using an ELISAPOD standard TMB kit (manufactured by Nacalai Tesque Inc.). Next, 50 µL of 1 mol/L sulfate was added to each well to terminate the reaction, and an absorbance for each well was measured at 450 nm by using a 96-well plate reader. The cells corresponding to the wells showing a high measurement value were selected as the high expression cell line of the humanized anti-hTfR antibody-hIDUA fusion protein. The high expression cell line of the humanized anti-hTfR antibody-hIDUA fusion protein obtained as described above was designated as a humanized anti-hTfR antibody-hIDUA expressing strain. The fusion protein of the humanized anti-hTfR antibody and hIDUA expressed by cell line was designated as the humanized anti-hTfR antibody-hIDUA fusion protein (humanized anti-hTfR antibody-hIDUA).

The obtained humanized anti-hTfR antibody-hIDUA expressing strain was suspended in a CD OptiCHO^{™} medium containing 10 mg/L of insulin, 16 µmol/L of thymidine, 100 µmol/L of hypoxanthine, 500 µg/mL of hygromycin B, 10 µg/mL of puromycin, 300 µmol/L of MSX, and 10% (v/v) DMSO, and then, was injected to a cryotube, and was stored in liquid nitrogen as seed cells.

### [Example 3] Culture of Humanized Anti-hTfR Antibody-hIDUA Expressing Strain

In order to acquire the humanized anti-hTfR antibody-hIDUA, the humanized anti-hTfR antibody-hIDUA expressing strain was cultured by the following method. The humanized anti-hTfR antibody-hIDUA expressing strain obtained in Example 2 was suspended in an approximately 170 L of a serum-free medium (a CD OptiCHO^{™} medium, manufactured by Thermo Fisher Scientific Inc.) adjusted to pH 6.9, which contained 10 mg/L of insulin, 16 µmol/L of thymidine, and 100 µmol/L of hypoxanthine such that a cell density was approximately 3 × 10⁵ cells/mL. 170 L of the cell suspension was transferred to a culture tank. The medium was stirred with an impeller at a speed of approximately 100 rpm, the dissolved oxygen saturation level of the medium was retained at approximately 30%, and the cells were cultured in a temperature range of 34°C to 37°C for approximately 10 days. During a culture period, the cell density, a cell viability, a medium glucose concentration, and a lactate concentration were monitored. In a case where the medium glucose concentration was 3.0 g/L or less, a glucose solution was immediately added to the medium such that the glucose concentration was 3.5 g/L. During the culture period, a feed solution (EFFICIENTFEED A+^{™}, manufactured by Thermo Fisher Scientific Inc.) was suitably added to the medium. After the completion of the culture, the medium was collected. The collected medium was filtered with Millistak+HC Pod Filter grade D0HC (manufactured by Merck & Co., Inc.), and was further filtered with Millistak+HC grade X0HC (manufactured by Merck & Co., Inc.) to obtain a culture supernatant containing the humanized anti-hTfR antibody-hIDUA. The culture supernatant was subjected to ultrafiltration by using Pellicon^{™} 3 Cassette w/Ultracel PLCTK Membrane (Pore Size: 30 kDa, Membrane Area: 1.14 m², manufactured by Merck & Co., Inc.), and a liquid volume was concentrated until the liquid volume was approximately 1/14. Next, the concentrate was filtered by using Opticap XL600 (0.22 µm, manufactured by Merck & Co., Inc.). The obtained solution was a concentrated culture supernatant.

### [Example 4] Purification of Humanized Anti-hTfR Antibody-hIDUA

0.25 volumes of 2 M arginine solution (pH 7.0) was added to the concentrated culture supernatant obtained in Example 3. The solution was loaded to a Capture Select^{™} CH1-XL column (Column Volume: approximately 3.1 L, Bed Height: approximately 20 cm, manufactured by Thermo Fisher Scientific Inc.) equilibrated with 4 column volumes of 25 mM MES buffer solution (pH 6.5) containing 400 mM arginine at a constant flow rate of 100 cm/hour such that the humanized anti-hTfR antibody-hIDUA was adsorbed in the column. The Capture Select^{™} CH1-XL column is an affinity column in which a ligand having properties to be specifically bound to a CH1 domain of an IgG antibody is fixed to a carrier.

Next, the column was washed with 5 column volumes of the same buffer solution by providing the solution at the same flow rate. Next, the column was further washed with 3 column volumes of 25 mM MES buffer solution (pH 6.5) by providing the solution at the same flow rate. Next, the humanized anti-hTfR antibody-hIDUA adsorbed in the column was eluted with 5 column volumes of 10 mM sodium acetate-HCl buffer solution (pH 3.5). The eluate was received in a container containing in advance 250 mM MES buffer solution (pH 6.0), and was immediately neutralized.

250 mM MES buffer solution (pH 6.5) was added to the eluate from the affinity column described above such that the pH of the eluate was adjusted to 6.0. Next, the eluate was filtered with Opticap XL600 (Pore Size: 0.22 µm, manufactured by Merck & Co., Inc.). The filtered solution was equilibrated with 5 column volumes of 50 mM MES buffer solution (pH 6.0) containing 15 mM NaCl, and was loaded to a Capto adhere column (Column Volume: approximately 1.5 L, Bed Height: approximately 10 cm, manufactured by GE Healthcare Inc.), which is a multimodal anion exchange column, at a constant flow rate of 300 cm/hour. A load liquid containing the humanized anti-hTfR antibody-hIDUA was collected. Since the Capto adhere contains N-benzyl-N-methyl ethanol amine as a ligand, the Capto adhere is a strong anion exchanger having selectivity based on electrostatic interaction, a hydrogen bond, hydrophobic interaction, and the like.

Next, the column was washed with 5 column volumes of the same buffer solution by providing the solution at the same flow rate, and the washing liquid was collected.

The load liquid and the washing liquid were loaded to a Capto MM column (Column Volume: approximately 3.1 L, Bed Height: approximately 20 cm, manufactured by GE Healthcare Inc.) equilibrated with 4 column volumes of 25 mM MES buffer solution (pH 6.5) containing 300 mM NaCl, which is a multimodal weak cation exchange column, at a constant flow rate of 200 cm/hour. Capto MMC is a weak cation exchanger having selectivity based on the hydrophobic interaction, the hydrogen bond formation, or the like.

Next, the column was washed with 5 column volumes of the same buffer solution by providing the solution at the same flow rate. Next, the humanized anti-hTfR antibody-hIDUA adsorbed in the weak cation exchange column was eluted with 10 column volumes of 25 mM MES buffer solution (pH 6.5) containing 1 M NaCl.

0.5 volumes of 20 mM citric acid buffer solution (pH 5.5) containing 0.8 mg/mL of NaCl and 75 mg/mL of sucrose was added to the eluate from the weak cation exchange column to adjust the pH to 5.8. Next, ultrafiltration was performed by using Pellicon^{™} 3 Cassette w/Ultracel PLCTK Membrane (Pore Size: 30 kDa, Membrane Area: 0.57 m², manufactured by Merck & Co., Inc.), and concentration was performed until the concentration of the humanized anti-hTfR antibody-hIDUA in the solution was approximately 30 mg/mL. Next, the concentrate was filtered by using Opticap XL600 (0.22 µm, manufactured by Merck & Co., Inc.).

The concentrate was loaded to a BioSEC column (Column Volume: approximately 9.4 L, Bed Height: 30 cm, manufactured by Merck & Co., Inc.) equilibrated with 1.5 column volumes of 20 mM citric acid buffer solution (pH 5.5) containing 0.8 mg/mL of NaCl and 75 mg/mL of sucrose, which is a size exclusion column, at a constant flow rate of 40 cm/hour, and the same buffer solution was provided at the same flow rate. In this case, an absorbance photometer for consecutively measuring the absorbance of the eluate was disposed on the flow path of the eluate from the size exclusion column, an absorbance at 280 nm was monitored, and a fraction showing an absorption peak at 280 nm was collected as a fraction containing the humanized anti-hTfR antibody-hIDUA to be the purified product of the humanized anti-hTfR antibody-hIDUA.

### [Example 5] Measurement of Collection Rate of Humanized Anti-hTfR Antibody-hIDUA in Each Purification Step

The loaded amount of the humanized anti-hTfR antibody-hIDUA and the collected amount of the humanized anti-hTfR antibody-hIDUA in the eluate in a purification step using the affinity column were measured by using the ELISA method described in Example 2. In addition, the loaded amount of the humanized anti-hTfR antibody-hIDUA and the collected amount of the humanized anti-hTfR antibody-hIDUA in the eluate in each of the other purification steps were calculated by measuring an absorbance at 280 nm with a spectrophotometer. The results are shown in Table 3. Originally, 17.5 g of the humanized anti-hTfR antibody-hIDUA, corresponding to approximately 74.0% of 23.7 g of the humanized anti-hTfR antibody-hIDUA contained in the culture supernatant, was collected as the purified product. Such results indicate that the purification method described in Example 4 is extremely efficient as a purification method of the humanized anti-hTfR antibody-hIDUA. Note that, in Table 3, a process collection rate (%) indicates a ratio of the amount of collected humanized anti-hTfR antibody-hIDUA to the amount of loaded humanized anti-hTfR antibody-hIDUA in each purification step, and the total collection rate (%) indicates a ratio of the amount of humanized anti-hTfR antibody-hIDUA collected in each purification step to the initial amount of the amount of humanized anti-hTfR antibody-hIDUA provided to the purification process.

**[Table 3]**

| (Table 3) Collection Rate of Humanized Anti-hTfR Antibody-hIDUA in Each Purification Step | | | | |
|---|---|---|---|---|
| Purification step | Humanized anti-hTfR antibody-hIDUA | | | |
| | Loaded amount (g) | Eluted amount (g) | Process collection rate (%) | Total collection rate (%) |
| Affinity column | 23.7 | 22.2 | 93.7 | 93.7 |
| Multimodal anion exchange column | 22.0 | 22.0 | 100.0 | 92.7 |
| Multimodal weak cation exchange column | 21.6 | 20.5 | 94.7 | 86.4 |
| Gel filtration column | 20.1 | 17.5 | 87.1 | 74.0 |

### [Example 6] Analysis of Purified Product of Humanized Anti-hTfR Antibody-hIDUA (Quantification of HCP)

The amount of host cell-derived protein (HCP) contained in the purified product of the humanized anti-hTfR antibody-hIDUA obtained in Example 4 was quantified by an ELISA method. First, 100 µL of an anti-CHO cell-derived protein antibody was added to each well of a 96-well plate (manufactured by Nunc Inc.), and was left to stand overnight to adsorb the antibody. After each well was washed three times, 200 µL of a blocking solution containing casein was added to each well, followed by shaking at 25°C for 60 minutes. After each well was washed three times, 100 µL of each of a solution (a sample solution) containing the purified product of the humanized anti-hTfR antibody-hIDUA or a HCP standard solution was added to each well, followed by shaking 25°C for 2 hours. After each well was washed three times, 100 µL of a biotinylated anti-CHO cell-derived protein antibody was added to each well, followed by shaking at 25°C for 60 minutes. After each well was washed three times, 100 µL of HRP-conjugated streptavidin (manufactured by Jackson Immuno Research Laboratories Inc.) was added, followed by shaking at 25°C for 60 minutes. After each well was washed three times, 100 µL of a TMB substrate solution was added to each well to develop a color at 25°C. As the TMB substrate solution, a mixture was used in which a TMB peroxidase substrate and a Peroxidase substrate solution B of a TMB microwell peroxidase substrate system (manufactured by Kirkegaard & Perry Laboratories Inc.) were mixed at the equal amount. After the color development, 100 µL of 6.75% phosphoric acid was added to each well to terminate the enzyme reaction, and an absorbance at 450 nm of each well was measured with a 96-well plate reader. A standard curve was prepared from the measurement value of the HCP standard solution, and the value of the sample solution was interpolated to the standard curve to quantify HCP contained in the purified product of the humanized anti-hTfR antibody-hIDUA. HCP contained in the purified produce of the humanized anti-hTfR antibody-hIDUA was quantified from the quantified value of HCP obtained as described above, and the quantified value of the purified product of the humanized anti-hTfR antibody-hIDUA measured by using the ELISA method described in Example 2. As a result thereof, it was found that the amount of HCP contained in the purified product of the humanized anti-hTfR antibody-hIDUA was approximately 35 ppm (that is, approximately 35 ng of HCP in 1 mg of the purified product of the humanized anti-hTfR antibody-hIDUA).

### [Example 7] Analysis of Purified Product of Humanized Anti-hTfR Antibody-hIDUA (Analysis of SE-HPLC)

ATSKgel G3000SW_{XL} column (Inner Diameter 7.8 mm × Height 30 cm, manufactured by Tosoh Corporation) was set in a UV/VIS detector (manufactured by SHIMADZU CORPORATION) of SPD-20AV, which is a LC-20A system. The column was equilibrated with 200 mM phosphoric acid buffer solution containing 5% 2-propanol and 20 mM NaCl. 10 µL of a solution containing the purified product of the humanized anti-hTfR antibody-hIDUA obtained in Example 4 at a concentration of 1.0 mg/mL was loaded to this column at a constant flow rate of 0.6 mL/minute, and the same buffer solution was further provided at the same flow rate. An elution profile prepared by measuring an absorbance at 215 nm is illustrated in FIG. 1. The obtained profile approximately indicates only a single peak derived from the humanized anti-hTfR antibody-hIDUA. However, a peak (a polymer peak in the drawing) derived from a polymer of the humanized anti-hTfR antibody-hIDUA detected prior to a main peak (a monomer peak in the drawing) was observed. From a ratio of the area of the polymer peak to the area of the whole peak, a ratio of the polymer to the whole humanized anti-hTfR antibody-hIDUA was calculated to be approximately 0.02%.

### [Example 8] Analysis of Purified Product of Humanized Anti-hTfR Antibody-hIDUA (Summary)

The analysis results of the purified product of the humanized anti-hTfR antibody-hIDUA indicate that the purified product of the humanized anti-hTfR antibody-hIDUA obtained in Example 4 contains almost no contaminants including HCP and the abundance ratio of the polymer is extremely low. That is, it is concluded that the purified product of the humanized anti-hTfR antibody-hIDUA has quality to the extent that the purified product can be used as medical drugs as it is, for example, as intravenously, intramuscularly, subcutaneously, intraperitonealy, intraarterialy or intralesionaly administered medicine.

### Industrial Applicability

According to the present invention, for example, it is possible to provide a fusion protein in which an antibody and a lysosomal enzyme are bound, in particular, a fusion protein in which an antibody and hIDUA are bound, purified to a purity to the extent that the fusion protein can be used as medical drugs as it is.

### Sequence Listing Free Text

SEQ ID NO:1 = Amino acid sequence of exemplified linker 1
SEQ ID NO:2 = Amino acid sequence of exemplified linker 2
SEQ ID NO:3 = Amino acid sequence of exemplified linker 3
SEQ ID NO:4 = Amino acid sequence of exemplified linker 4
SEQ ID NO:5 = Amino acid sequence of human transferrin receptor
SEQ ID NO:6 = Amino acid sequence 1 of human IDUA
SEQ ID NO:7 = Amino acid sequence 2 of human IDUA
SEQ ID NO:8 = Amino acid sequence 1 of light chain CDR1 of anti-hTfR antibody
SEQ ID NO:9 = Amino acid sequence 2 of light chain CDR1 of anti-hTfR antibody
SEQ ID NO: 10 = Amino acid sequence 1 of light chain CDR2 of anti-hTfR antibody
SEQ ID NO: 11 = Amino acid sequence 2 of light chain CDR2 of anti-hTfR antibody
SEQ ID NO: 12 = Amino acid sequence 1 of light chain CDR3 of anti-hTfR antibody
SEQ ID NO: 13 = Amino acid sequence 1 of heavy chain CDR1 of anti-hTfR antibody
SEQ ID NO: 14 = Amino acid sequence 2 of heavy chain CDR1 of anti-hTfR antibody
SEQ ID NO: 15 = Amino acid sequence 1 of heavy chain CDR2 of anti-hTfR antibody
SEQ ID NO: 16 = Amino acid sequence 2 of heavy chain CDR2 of anti-hTfR antibody
SEQ ID NO: 17 = Amino acid sequence 1 of heavy chain CDR3 of anti-hTfR antibody
SEQ ID NO: 18 = Amino acid sequence 2 of heavy chain CDR3 of anti-hTfR antibody
SEQ ID NO: 19 = Amino acid sequence of heavy chain framework region 3 of anti-hTfR antibody
SEQ ID NO:20 = Amino acid sequence of variable region of light chain of anti-hTfR antibody
SEQ ID NO:21 = Amino acid sequence of variable region of heavy chain of anti-hTfR antibody
SEQ ID NO:22 = Amino acid sequence of light chain of anti-hTfR antibody
SEQ ID NO:23 = Amino acid sequence of Fab heavy chain of anti-hTfR antibody
SEQ ID NO:24 = Primer Hyg-Sfi5', synthetic sequence
SEQ ID NO:25 = Primer Hyg-BstX3', synthetic sequence
SEQ ID NO:26 = Base sequence encoding amino acid sequence of light chain of anti-hTfR antibody, synthetic sequence
SEQ ID NO:27 = Amino acid sequence of fusion protein of Fab heavy chain of humanized anti-hTfR antibody and human IDUA
SEQ ID NO:28 = Base sequence including gene encoding amino acid sequence of fusion protein of Fab heavy chain of humanized anti-hTfR antibody and human IDUA, synthetic sequence
SEQ ID NO:29 = Primer IRES5', synthetic sequence
SEQ ID NO:30 = Primer IRES3', synthetic sequence
SEQ ID NO:31 = Primer mPGKP5', synthetic sequence
SEQ ID NO:32 = Primer mPGKP3', synthetic sequence
SEQ ID NO:33 = Primer GS5', synthetic sequence
SEQ ID NO:34 = Primer GS3', synthetic sequence
SEQ ID NO:35 = Primer puro5', synthetic sequence
SEQ ID NO:36 = Primer puro3', synthetic sequence
SEQ ID NO:37 = Primer SV40polyA5', synthetic sequence
SEQ ID NO:38 = Primer SV40polyA3', synthetic sequence
SEQ ID NO:39 = Primer mIRES-GS5', synthetic sequence
SEQ ID NO:40 = primer mIRES-GS3', synthetic sequence
SEQ ID NO:41 = CMVE-EF-1αp-IFNβMAR, synthetic sequence
SEQ ID NO:42 = IRES-HygroR-mPGKpA, synthetic sequence

## Claims

1. A method for producing a fusion protein in which an antibody and a human lysosomal enzyme are fused, comprising:
(a) culturing mammalian cells producing the fusion protein in a serum-free medium to secrete the fusion protein in a culture medium;
(b) collecting a culture supernatant by removing the mammalian cells from the culture medium; and
(c) purifying the fusion protein from the culture supernatant by using a column chromatography using a material to which a substance having affinity for the antibody is bound as a solid phase, an anion exchange column chromatography, a cation exchange column chromatography, and a size exclusion column chromatography.

2. The method for producing a fusion protein according to claim 1,
wherein the column chromatography using the material to which the substance having affinity for the antibody is bound as a solid phase, the anion exchange column chromatography, the cation exchange column chromatography, and the size exclusion column chromatography are used in this order.

3. The method for producing a fusion protein according to claim 1 or 2,
wherein the substance having affinity for the antibody has affinity for a CH₁ region of a heavy chain of the antibody.

4. The method for producing a fusion protein according to any one of claims 1 to 3,
wherein an anion exchanger used in the anion exchange column chromatography is a strong anion exchanger.

5. The method for producing a fusion protein according to any one of claims 1 to 4,
wherein a cation exchanger used in the cation exchange column chromatography is a weak cation exchanger.

6. The method for producing a fusion protein according to any one of claims 1 to 5,
wherein the antibody fused with the human lysosomal enzyme is a humanized antibody or a human antibody.

7. The method for producing a fusion protein according to any one of claims 1 to 5,
wherein the antibody fused with the human lysosomal enzyme is a humanized antibody.

8. The method for producing a fusion protein according to any one of claims 1 to 7,
wherein the antibody fused with the human lysosomal enzyme recognizes a molecule present on a surface of vascular endothelial cells as an antigen.

9. The method for producing a fusion protein according to claim 8,
wherein the molecule present on the surface of the vascular endothelial cells is selected from the group consisting of a transferrin receptor (TfR), an insulin receptor, a leptin receptor, a lipoprotein receptor, an IGF receptor, OATP-F, an organic anion transporter, MCT-8, a monocarboxylic acid transporter, and a Fc receptor.

10. The method for producing a fusion protein according to claim 8,
wherein the vascular endothelial cells are cerebral vascular endothelial cells.

11. The method for producing a fusion protein according to claim 10,
wherein a molecule present on a surface of the cerebral vascular endothelial cells is selected from the group consisting of a transferrin receptor (TfR), an insulin receptor, a leptin receptor, a lipoprotein receptor, an IGF receptor, OATP-F, an organic anion transporter, MCT-8, and a monocarboxylic acid transporter.

12. The method for producing a fusion protein according to any one of claims 8 to 11,
wherein the vascular endothelial cells are human vascular endothelial cells.

13. The method for producing a fusion protein according to any one of claims 1 to 12,
wherein the antibody is an anti-human transferrin receptor antibody,
in a variable region of the heavy chain of the antibody, CDR1 includes an amino acid sequence of SEQ ID NO: 13 or SEQ ID NO: 14, CDR2 includes an amino acid sequence of SEQ ID NO: 15 or SEQ ID NO: 16, and CDR3 includes an amino acid sequence of SEQ ID NO: 17 or SEQ ID NO: 18, and
in a variable region of a light chain of the antibody, CDR1 includes an amino acid sequence of SEQ ID NO:8 or SEQ ID NO:9, CDR2 includes an amino acid sequence of SEQ ID NO: 10 or SEQ ID NO: 11, or an amino acid sequence Lys-Val-Ser, and CDR3 includes an amino acid sequence of SEQ ID NO: 12.

14. The method for producing a fusion protein according to claim 13,
wherein a framework region 3 of the heavy chain of the antibody includes an amino acid sequence of SEQ ID NO: 19.

15. The method for producing a fusion protein according to claim 14,
wherein the variable region of the heavy chain in the antibody includes an amino acid sequence of SEQ ID NO:21.

16. The method for producing a fusion protein according to claim 13 or 14,
wherein in the variable region of the heavy chain, the antibody includes an amino acid sequence having 80% or more of identity with an amino acid sequence of SEQ ID NO:21,
CDR1 includes an amino acid sequence of SEQ ID NO: 13 or SEQ ID NO: 14,
CDR2 includes an amino acid sequence of SEQ ID NO: 15 or SEQ ID NO: 16,
CDR3 includes an amino acid sequence of SEQ ID NO: 17 or SEQ ID NO: 18, and
the framework region 3 includes an amino acid sequence of SEQ ID NO: 19.

17. The method for producing a fusion protein according to claim 13 or 14,
wherein in the variable region of the heavy chain, the antibody includes an amino acid sequence having 90% or more of identity with an amino acid sequence of SEQ ID NO:21,
CDR1 includes an amino acid sequence of SEQ ID NO: 13 or SEQ ID NO: 14,
CDR2 includes an amino acid sequence of SEQ ID NO: 15 or SEQ ID NO: 16,
CDR3 includes an amino acid sequence of SEQ ID NO: 17 or SEQ ID NO: 18, and
the framework region 3 includes an amino acid sequence of SEQ ID NO: 19.

18. The method for producing a fusion protein according to claim 13 or 14,
wherein in the variable region of the heavy chain, the antibody includes an amino acid sequence obtained by modifying an amino acid sequence of SEQ ID NO:21 with substitution, deletion, or addition of 1 to 5 amino acids,
CDR1 includes an amino acid sequence of SEQ ID NO: 13 or SEQ ID NO: 14,
CDR2 includes an amino acid sequence of SEQ ID NO: 15 or SEQ ID NO: 16,
CDR3 includes an amino acid sequence of SEQ ID NO: 17 or SEQ ID NO: 18, and
the framework region 3 includes an amino acid sequence of SEQ ID NO: 19.

19. The method for producing a fusion protein according to claim 13 or 14,
wherein in the variable region of the heavy chain, the antibody includes an amino acid sequence obtained by modifying an amino acid sequence of SEQ ID NO:21 with substitution, deletion, or addition of 1 to 3 amino acids,
CDR1 includes an amino acid sequence of SEQ ID NO: 13 or SEQ ID NO: 14,
CDR2 includes an amino acid sequence of SEQ ID NO: 15 or SEQ ID NO: 16,
CDR3 includes an amino acid sequence of SEQ ID NO: 17 or SEQ ID NO: 18, and
the framework region 3 includes an amino acid sequence of SEQ ID NO: 19.

20. The method for producing a fusion protein according to any one of claims 13 to 19,
wherein the variable region of the light chain of the antibody includes an amino acid sequence of SEQ ID NO:20.

21. The method for producing a fusion protein according to any one of claims 13 to 19,
wherein in the variable region of the light chain, the antibody includes an amino acid sequence having 80% or more of identity with an amino acid sequence of SEQ ID NO:20,
CDR1 includes an amino acid sequence of SEQ ID NO:8 or SEQ ID NO:9,
CDR2 includes an amino acid sequence of SEQ ID NO: 10 or SEQ ID NO: 11, or an amino acid sequence Lys-Val-Ser, and
CDR3 includes an amino acid sequence of SEQ ID NO: 12.

22. The method for producing a fusion protein according to any one of claims 13 to 19,
wherein in the variable region of the light chain, the antibody includes an amino acid sequence having 90% or more of identity with an amino acid sequence of SEQ ID NO:20,
CDR1 includes an amino acid sequence of SEQ ID NO:8 or SEQ ID NOV,
CDR2 includes an amino acid sequence of SEQ ID NO: 10 or SEQ ID NO: 11, or an amino acid sequence Lys-Val-Ser, and
CDR3 includes an amino acid sequence of SEQ ID NO: 12.

23. The method for producing a fusion protein according to any one of claims 13 to 19,
wherein in the variable region of the light chain, the antibody includes an amino acid sequence obtained by modifying an amino acid sequence of SEQ ID NO:20 with substitution, deletion, or addition of 1 to 5 amino acids,
CDR1 includes an amino acid sequence of SEQ ID NO:8 or SEQ ID NOV,
CDR2 includes an amino acid sequence of SEQ ID NO: 10 or SEQ ID NO: 11, or an amino acid sequence Lys-Val-Ser, and
CDR3 includes an amino acid sequence of SEQ ID NO: 12.

24. The method for producing a fusion protein according to any one of claims 13 to 19,
wherein in the variable region of the light chain, the antibody includes an amino acid sequence obtained by modifying an amino acid sequence of SEQ ID NO:20 with substitution, deletion, or addition of 1 to 3 amino acids,
CDR1 includes an amino acid sequence of SEQ ID NO:8 or SEQ ID NO:9,
CDR2 includes an amino acid sequence of SEQ ID NO: 10 or SEQ ID NO: 11, or an amino acid sequence Lys-Val-Ser, and
CDR3 includes an amino acid sequence of SEQ ID NO: 12.

25. The method for producing a fusion protein according to claim 15,
wherein the heavy chain of the antibody includes an amino acid sequence of SEQ ID NO:23.

26. The method for producing a fusion protein according to claim 20 or 25,
wherein the light chain of the antibody includes an amino acid sequence of SEQ ID NO:22.

27. The method for producing a fusion protein according to any one of claims 1 to 26,
wherein the antibody is Fab, F(ab')₂, or F(ab').

28. The method for producing a fusion protein according to any one of claims 1 to 27,
wherein the human lysosomal enzyme in the fusion protein is bound to a C-terminal side or an N-terminal side of the light chain of the antibody.

29. The method for producing a fusion protein according to claim 28,
wherein the human lysosomal enzyme in the fusion protein is bound to the C-terminal side or the N-terminal side of the light chain directly or via a linker.

30. The method for producing a fusion protein according to claim 28,
wherein the human lysosomal enzyme in the fusion protein is bound to a C-terminal side or an N-terminal side of the heavy chain via a linker.

31. The method for producing a fusion protein according to claim 29 or 30,
wherein a linker sequence is peptide including 1 to 50 amino acid residues.

32. The method for producing a fusion protein according to claim 31,
wherein the linker is peptide including an amino acid sequence selected from the group consisting of single glycine, single serine, an amino acid sequence Gly-Ser, an amino acid sequence Gly-Gly-Ser, an amino acid sequence of SEQ ID NO:1, an amino acid sequence of SEQ ID NO:2, an amino acid sequence of SEQ ID NO:3, an amino acid sequence of SEQ ID NO:4, and an amino acid sequence including 1 to 10 consecutive amino acid sequences.

33. The method for producing a fusion protein according to any one of claims 1 to 32,
wherein the human lysosomal enzyme in the fusion protein is human α-L-iduronidase.

34. The method for producing a fusion protein according to claim 33,
wherein the antibody in the fusion protein is Fab,
the light chain of the antibody includes an amino acid sequence of SEQ ID NO:22, and
the heavy chain of the antibody is bound to the human α-L-iduronidase via an amino acid sequence of SEQ ID NO:4 on the C-terminal side such that the fusion protein forms an amino acid sequence of SEQ ID NO:27.

35. The method for producing a fusion protein according to claim 33,
wherein the antibody in the fusion protein is Fab,
the light chain of the antibody includes an amino acid sequence of SEQ ID NO:22, and
the heavy chain of the antibody includes an amino acid sequence of SEQ ID NO:23, and is bound to the human α-L-iduronidase including an amino acid sequence of SEQ ID NO:6 via a linker including an amino acid sequence of SEQ ID NO:4 on the C-terminal side.

36. The method for producing a fusion protein according to claim 33,
wherein the human α-L-iduronidase includes an amino acid sequence of SEQ ID NO:6 or 7.
